# FASCICULE DE BREVET EUROPEEN

(11) **EP 3 752 594 B1**
(45) Date de publication et mention de la délivrance du brevet: **06.04.2022**
(21) Numéro de dépôt: 19708410.6
(22) Date de dépôt: 15.02.2019
(51) Int. Cl.: C12M 1/12, C12N 5/00, C08L 33/20, D01D 5/00, D01D 10/02, D01F 6/18

(54) **RESEAU TRIDIMENSIONNEL BIOCOMPATIBLE ET SON UTILISATION EN TANT QUE SUPPORT DE CELLULES**
BIOKOMPATIBLES DREIDIMENSIONALES NETZWERK UND SEINE VERWENDUNG ALS ZELLTRÄGER
BIOCOMPATIBLE THREE-DIMENSIONAL NETWORK AND USE THEREOF AS CELL SUPPORT

(30) Priorité: 16.02.2018 FR 1851324
(43) Date de publication de la demande: 23.12.2020
(73) Titulaire: Université de Montpellier, 34090 Montpellier (FR); Institut National de la Santé et de la Recherche Médicale, 75013 Paris 13 (FR); Centre National de la Recherche Scientifique, 75016 Paris (FR); Ecole Nationale Supérieure de Chimie de Montpellier, 34090 Montpellier (FR)
(72) Inventeur: CORNU, David, 34730 PRADES-LE-LEZ (FR); BAKALARA, Norbert, 34380 SAINT-MARTIN-DE-LONDRES (FR); MARHUENDA, Emilie, 34090 MONTPELLIER (FR); SALEH, Ali, Jarjouh, Nabatieh (LB)
(74) Mandataire: IPAZ
(86) Numéro de dépôt international: PCT/EP2019/053883
(87) Numéro de publication internationale: WO 2019/158724

(56) Documents cités:
- JIAN-FENG PAN ET AL: "Preparation and Characterization of Electrospun PLCL/Poloxamer Nanofibers and Dextran/Gelatin Hydrogels for Skin Tissue Engineering", PLOS ONE, vol. 9, no. 11, 18 novembre 2014 (2014-11-18), page e112885, XP055516341, DOI: 10.1371/journal.pone.0112885
- MAURO SANTOS DE OLIVEIRA JUNIOR ET AL: "A statistical approach to evaluate the oxidative process of electrospun polyacrylonitrile ultrathin fibers : ARTICLE", JOURNAL OF APPLIED POLYMER SCIENCE, vol. 134, no. 43, 15 novembre 2017 (2017-11-15), page 45458, XP055513956, ISSN: 0021-8995, DOI: 10.1002/app.45458
- CHING-IUAN SU ET AL: "Optimum heat treatment conditions for PAN-based oxidized electrospun nonwovens", FIBERS AND POLYMERS, THE KOREAN FIBER SOCIETY, HEIDELBERG, vol. 13, no. 1, 18 février 2012 (2012-02-18), pages 38-43, XP035017209, ISSN: 1875-0052, DOI: 10.1007/S12221-012-0038-7
- NURAY KIZILDAG: "Composite Nanofibers of Polyacrylonitrile (PAN) and Amino-functionalized Carbon Nanotubes Electrospun from Dimethylsulfoxide", MARMARA UNIVERSITY JOURNAL OF SCIENCE, vol. 27, no. 3, 1 janvier 2015 (2015-01-01), XP055516349, ISSN: 2146-5150, DOI: 10.7240/mufbed.13275
- YÖRDEM ET AL: "Effects of electrospinning parameters on polyacrylonitrile nanofiber diameter: An investigation by response surface methodology", MATERIALS AND DESIGN, LONDON, GB, vol. 29, no. 1, 18 septembre 2007 (2007-09-18), pages 34-44, XP022255910, ISSN: 0261-3069, DOI: 10.1016/J.MATDES.2006.12.013
- LI W-J ET AL: "ELECTROSPUN NANOFIBROUS STRUCTURE: A NOVEL SCAFFOLD FOR TISSUE ENGINEERING", JOURNAL OF BIOMEDICAL MATERIALS RESEARCH, WILEY, NEW YORK, NY, US, vol. 60, no. 4, 15 juin 2002 (2002-06-15), pages 613-621, XP001183765, ISSN: 0021-9304, DOI: 10.1002/JBM.10167

## Description

La présente invention se situe dans le domaine des dispositifs tridimensionnels biocompatibles.

L'invention a pour objet un réseau tridimensionnel (3D) de fibres biocompatibles, et sa forme intégrée dans un dispositif, elle a également pour objet un procédé de fabrication d'un tel réseau de fibres. L'invention a également pour objet les utilisations dudit réseau en tant que support de cellules pour l'étude de comportements cellulaires au sein d'un environnement 3D, notamment la survie, la prolifération, la migration et l'invasivité des cellules.

### ETAT DE L'ART

Des réseaux et dispositifs simulant en deux dimensions les propriétés physiques des conditions de migration cellulaire *in vivo* sont couramment utilisés et commercialisés. Plusieurs types de réseaux ou dispositifs 3Ds sont en cours de développement ; les réseaux actuellement connus sont constitués par un matériau tel que les tissus dé-cellularisés, les couches de cellules, les hydrogels, les fibres électrofilées et les éponges et présentent des inconvénients divers selon la nature du matériau utilisé.

Shogolu et al. (« Recreating complex pathophysiologies in vitro with extracellular matrix surrogates for anticancer therapeutics screening », Drug Discov. Today, vol. 21, N° 9, p. 1521-31, 2016) décrit différent dispositifs 3D mimant le microenvironnement *in vitro* des tumeurs et cite leurs inconvénients : les tissus dé-cellularisés intacts sont délicats à obtenir et ne permettent pas une standardisation et une reproductibilité suffisante, les monocouches de cellules, les hydrogels peuvent présenter une importante cytotoxicité, les fibres électrofilées ne permettent pas une bonne infiltration cellulaire, les éponges n'autorisent pas un contrôle et de la rigidité, la dégradation ou de la porosité du réseau.

Bui et al. (« Brain tumor genetic modification yields increased resistance to paclitaxel in physical confinement », Nature Sci. Reports 6:26134, 2016) décrit un dispositif microfluidique en polydimethylsiloxane (PDMS) générant trois degrés différents de confinement physique : un confinement étroit (5x5 µm), large (15x15 µm) et 2D (hauteur de 150µm). Ce dispositif est recouvert de laminine avant usage. Ce système est rigide et les interactions des cellules avec les faces du support sont planes comme en 2D.

Akhmanova et al. (« Physical, spatial and molecular aspects of extracellular matrix of in vivo niches and artificial scaffolds relevant to stem cells research », Stem Cells International, Vol. 2015, article ID 167025, 2015) décrit des dispositifs comprenant des fibres électrofilées constituées de polyvinyl alcool (PVA), polydimethylsiloxane (PDMS), polyethylène glycol dimethacrylate (PEGdma), polyethylène oxyde (PEO), polycaprolactone (PCL), polyuréthane (PU), polyacrylamide (PAA).

Lee et al. (« Plasma treated flexible aminoclay-decorated electrospun nanofibers for neural stem cell self-renewal », J. Nanosci. Nanotechnol., Feb 16(2) : 1392-5, 2016) décrit des nanofibres obtenues par électrofilage de polyacrylonitrile (PAN) et comprenant des aminoargiles de magnésium ou de fer.

Liu et al. (« Electrospun polyacrylonitrile-based nanofibers maintain embryonic stem cell stemness via TGF-beta signaling » J Biomed Nanotechnol. Apr;12(4):732-42, 2016) décrit un dispositif de culture cellulaire constitué par des fibres de PAN biocompatible traitées par électrofilage.

Mahmoudifard M. et al. (« The different fate of satellite cells on conductive composite electrospun nanofibers with graphene and graphene oxide nanosheets", Biomed Mater. Mar 10; 11(2) 2016) décrit un réseau de fibres de polyaniline (PANI) et de PAN, additionné de nano-feuillets de graphène ou de graphène oxyde.

Jain et al. (« Biomaterials for liver tissue engineering" Hepatol Int. Apr;8(2): 185-97, 2014) étudie la cytocompatibilité *in vitro* du polyacrylonitrile (PAN), sous la forme de nanofibres de carbone ou de films fins.

US 2016/0377600 décrit un réseau 3D synthétisé par l'électrofilage d'une composition bifonctionnelle comprenant un polymère et des protéines. Après électrofilage, le réseau est directement placé dans une chambre à vide et conservé en atmosphère sèche avant utilisation.

US 2016/0355780 décrit un microenvironnement 3D adapté à la culture cellulaire, préparé par la combinaison d'au moins un polymère, naturel ou synthétique, et d'un motif peptidique activateur de l'intégrine. Ledit réseau 3D est obtenu par l'électrofilage d'une composition bifonctionnelle. Après électrofilage, le réseau est directement placé dans une chambre à vide et conservé en atmosphère sèche avant utilisation.

US 2012/0040581 a pour objet une méthode particulière de production de réseaux de nanofibres comprenant l'utilisation de moules prédéfinis.

Jian-Feng Pan et al. (PLOS ONE, vol. 9, no. 11, 2014) décrit la préparation et la caractérisation de réseaux 3D de fibres de Poly(e-caprolactone-co-lactide)/poloxàmere, associés à un hydrogel sans addition d'agent réticulant.

Les réseaux constitués de fibres électrofilées actuellement connus se caractérisent par une faible infiltration et croissance cellulaires, une distribution cellulaire non uniforme et par le fait que les cellules y migrent uniquement de façon indépendante. De plus, ces matériaux peuvent présenter une certaine toxicité vis-à-vis des cellules et ne permettent pas une analyse complète, notamment au niveau protéomique et fonctionnel, des cellules en migration. En effet, lorsque les réseaux sont constitués de collagène, l'analyse des protéines cellulaires est perturbée par le relargage lors de l'extraction protéique de protéines issues de ces réseaux. L'opacité de matériaux tels le poly-caprolactone nécessite l'emploi spécifique de microscopes bi-photoniques pour caractériser les cellules. Enfin, une éventuelle modulation des propriétés mécaniques d'un réseau 3D de l'état de l'art ne peut être réalisée indépendamment d'une modulation chimique de ce réseau, et réciproquement, ce qui limite les possibilités d'adaptation à différents types cellulaires.

Les inventeurs ont conçu et réalisé un réseau 3D constitué de fibres obtenues par électrofilage d'une solution de polymère de type acrylique, en particulier de polyacrylonitrile (PAN), auxquelles est appliqué un traitement thermique, réalisé à une température supérieure à 40°C et inférieure à 400°C, qui conduit à l'aromatisation et à la réticulation des fibres. De façon surprenante, ledit réseau présente des propriétés mécaniques très proches de l'environnement *in vivo* de cellules, et des propriétés de biocompatibilité, de non-toxicité et de support d'adhésion pour lesdites cellules. De plus, ledit réseau présente un caractère infusible, des propriétés optiques de fluorescence et des fonctions chimiques résiduelles en surface permettant son éventuelle fonctionnalisation ultérieure. Les propriétés autofluorescentes dudit réseau de fibres rend aisée la visualisation des cellules au sein du réseau, notamment par immunofluorescence ou mesure de l'efflux de calcium cellulaire mesurable par microscopie multiphoton, microscopie à épifluorescence ou un microscopie confocale, en utilisant les indicateurs marqués et un logiciel d'analyse approprié, bien connus de l'homme du métier. La possibilité de fonctionnalisation ultérieure du réseau rend possible l'étude et la caractérisation reproductible de comportements cellulaires au sein d'un environnement 3D, notamment la survie, la prolifération, la migration et l'invasivité des cellules.

Un réseau 3D selon l'invention présente l'avantage de pouvoir subir, de façon indépendante, la modulation de ses propriétés mécaniques et/ou de ses propriétés chimiques. Les propriétés mécaniques du réseau peuvent être modifiées par exemple par l'ajout de nanotubes de carbone qui augmentent sa rigidité. Les propriétés chimiques sont modulables par la fonctionnalisation de la surface des fibres, optimisant le mimétisme de l'interface substrat-cellules.

De plus, un réseau selon l'invention est caractérisé par sa non-cytotoxicité, sa stabilité (pas de relargage de composés lors de l'extraction de protéines des cellules présentes au sein du réseau) et par une élasticité pouvant être proche d'un tissu dans des conditions physiologiques et pathologiques ((« Tissue Stiffness Dictates Development, Homeostasis, and Disease Progression » Organogenesis. 11(1): 1-15, 2015), cette élasticité est par ailleurs modulable pour atteindre 1260 kPa au moins après l'addition de nanotubes de carbone. Un réseau selon l'invention permet donc de mimer au plus près l'environnement biologique des cellules.

Selon l'éventuelle fonctionnalisation des fibres et la nature de cette fonctionnalisation, un réseau 3D selon l'invention permet l'étude de la migration de différents types cellulaires. Le dépôt en surface des fibres de poly-D-Lysine et de laminine permet par exemple l'étude de la migration individuelle des cellules souches de glioblastome (GSC) alors qu'elles se déplacent de manière collective sur des fibres sans revêtement.

Les inventeurs ont montré que, lorsqu'elles sont mises en contact avec un réseau 3D selon l'invention, des cellules présentent des comportements migratoires similaires aux comportements migratoires décrits *in vivo* et qu'elles s'infiltrent au sein du réseau avec une répartition uniforme.

Enfin, ce réseau selon l'invention permet la visualisation et la mesure reproductible en microscopie des comportements cellulaires, notamment les comportements migratoires. Il permet également l'extraction de protéines analysables par western blot et la possibilité d'analyses complètes, incluant le transcriptome, le protéome et l'étude métabolomique, etc. Les fibres présentent une véritable facilité d'utilisation, peuvent être produites rapidement en grande quantité et leur fabrication est peu coûteuse.

Un réseau 3D selon l'invention est susceptible de s'intégrer de façon simple dans un dispositif de type « plaque multipuits » pour des études analytiques ou des études de screening, selon la nature du dispositif choisi.

L'invention a donc pour premier objet un réseau tridimensionnel infusible de fibres de polymère réticulé caractérisé en ce que le diamètre desdites fibres est compris entre 0,1 et 1,5 µm, la taille des interstices entre les fibres est comprise entre 0,1 et 50 µm² et la rigidité du réseau est caractérisée par un module d'élasticité compris entre 0,01 et 10 000 kPa. Ce réseau est susceptible d'être intégré au sein d'un dispositif.

L'invention a pour second objet un procédé de préparation d'un réseau tridimensionnel de fibres de polymère réticulé selon l'invention, ce procédé comprenant une étape de synthèse par électrofilage d'un réseau 3D de fibres, suivie d'une étape de traitement thermique du réseau 3D synthétisé, à une température supérieure à 40°C et inférieure à 400 °C, de préférence à une température supérieure à 200 °C et inférieure à 300 °C.

L'invention a pour troisième objet l'utilisation d'un réseau 3D de fibres de polymère réticulé selon l'invention en tant que support de cellules, ce support cellulaire étant utilisé *in vitro* pour la culture de cellules et l'étude de comportements cellulaires, notamment leur survie, leur prolifération, leur migration et leur invasivité. Les cellules étudiées sont notamment des cellules de glioblastome.

Cette utilisation comprend notamment la visualisation du comportement de cellules cultivées sur un réseau selon l'invention, par exemple par microscopie et l'analyse biochimique des cellules par toute méthode connue de l'homme du métier.

Enfin, l'invention a pour quatrième objet un dispositif de test, comme par exemple une plaque multi-puits ou une boîte de Petri, dans lequel est inséré un réseau 3D de fibres selon l'invention, découpé aux dimensions requises et inclus dans ledit dispositif, et son utilisation pour des applications analytiques.

D'autres caractéristiques, avantages et modes d'application des objets de l'invention sont détaillés dans la description ci-dessous, donnée à titre illustratif et non limitatif. Dans la suite du texte, les expressions « compris entre ... et ... » signifie que les bornes sont incluses.

### DESCRIPTION DETAILLEE

L'invention a pour premier objet un réseau tridimensionnel de fibres de polymère de type acrylique réticulé caractérisé en ce que :
- le diamètre des dites fibres est compris entre 0,1 et 1,5 µm, de préférence entre 0,3 et 1 µm, et plus préférentiellement entre 0,6 et 0,8 µm,
- la taille des interstices entre lesdites fibres est comprise entre 0,1 et 50 µm², de préférence entre 0,5 et 10 µm², et plus préférentiellement entre 1 et 2 µm², et
- la rigidité dudit réseau est caractérisée par un module d'élasticité compris entre 0,01 et 10 000 kPa, de préférence entre 0,1 et 10 000 kPa, de préférence entre 0,1 et 1 000 kPa, plus préférentiellement entre 0,1 et 300 kPa.

Par « fibres de polymère réticulé » on entend un matériau fibreux comprenant un polymère réticulé, ledit polymère réticulé étant obtenu par l'application d'un traitement thermique ou optique approprié à un polymère préalablement organisé sous la forme de fibres. Sous l'effet dudit traitement thermique ou optique, le polymère adopte une organisation structurale 3D particulière pour conduire à un polymère réticulé sans perdre sa morphologie fibreuse. La réticulation est définie comme la formation d'un réseau tridimensionnel par pontage des chaines polymériques. La nature du réseau dépend de la sollicitation thermique ou optique. Les procédés de synthèse de fibres de polymère sont bien connus de l'homme du métier, parmi ceux-ci on peut citer l'électrofilage. Les procédés de réticulation d'un polymère plastique par un traitement thermique ou optique sont également bien connus de l'homme du métier. Parmi les procédés thermiques de réticulation, l'application d'un traitement pendant au moins 10 minutes à une température comprise entre 40 et 400°C est connu pour conduire à l'aromatisation des fibres polymère et leur réticulation. Parmi les procédés optiques de réticulation, on peut citer notamment la photoréticulation UV.

Un réseau 3D de fibres de polymère réticulé selon l'invention est biocompatible, infusible et, de préférence, insoluble dans les solvants usuels utilisés en biologie, tels que notamment l'eau, les alcools ou le DMSO.

Par « biocompatible » on entend un support qui n'induit pas d'effet cytostatique et/ou cytotoxique vis-à-vis des cellules avec lequel il est en contact. Un réseau 3D selon l'invention est également de préférence non biodégradable au contact desdites cellules.

Dans un réseau 3D selon l'invention, ledit polymère réticulé est obtenu à partir :
- d'une solution de polymère comprenant : au moins 10% d'acrylique tel que le polyacrylonitrile (PAN) ou le polyméthacrylate de méthyle (PMMA) ; ou au moins 10 % d'un polyamide, par exemple le polyhexaméthylène adipamide (PA 6.6 ou nylon), ou au moins 10 % d'un autre motif réticulable, ou
- une solution de co-polymère ou de mélange comprenant au moins 10% du copolymère styrène-acrylonitrile (SAN), ou au moins 10 % d'un copolymère ou mélange tel que l'Acrylonitrile-Butadiène-Styrène (ABS), le butadiène-acrylonitrile (NBR), ou au moins 10% d'un autre motif réticulable.

Par « réseau 3D de fibres de polymère réticulé» on entend un réseau constitué de fibres dont au moins l'enveloppe, c'est-à-dire la partie superficielle, est constituée d'un matériau comprenant au moins 10% de polymère réticulé, ce pourcentage étant exprimé en pourcentage massique dudit matériau. Le cœur desdites fibres peut être constitué par tout matériau approprié, identique ou différent du matériau constituant l'enveloppe des fibres. Selon cet aspect, au moins l'enveloppe desdites fibres est constituée d'un matériau comprenant au moins 10 %, au moins 20 %, au moins 30 %, au moins 40 %, au moins 50 %, au moins 60 %, au moins 70 %, au moins 80 % ou au moins 90 % d'un polymère réticulé, ce pourcentage étant exprimé en pourcentage massique du matériau constituant la surface desdites fibres. Un réseau 3D selon l'invention dans lequel le cœur et l'enveloppe des fibres sont constitués de matériaux différents peut notamment être obtenu par un procédé d'électrofilage coaxial, dans lequel les polymères constituant l'enveloppe et le cœur desdites fibres sont projetés simultanément.

Selon un mode de réalisation particulier, l'enveloppe et le coeur des fibres d'un réseau 3D selon l'invention sont constituées d'un matériau comprenant au moins 10 %, au moins 20 %, au moins 30 %, au moins 40 %, au moins 50 %, au moins 60 %, au moins 70 %, au moins 80 % ou au moins 90 % d'un polymère réticulé, ce pourcentage étant exprimé en pourcentage massique du matériau constituant la surface desdites fibres.

Selon un mode de réalisation particulier, ledit polymère de type acrylique réticulé est obtenu à partir d'un polymère comprenant au moins 10% d'un polymère choisi parmi : les polymères de type acrylique, tel que notamment le polyacrylonitrile (PAN), et les polymères de type polyamide, lesdits polymères étant présents sous la forme de polymère ou de copolymère. Plus particulièrement, un réseau 3D selon l'invention est caractérisé en ce que polymère réticulé est obtenu à partir d'une solution de polymère de type acrylique, de préférence une solution comprenant au moins 10% de polymère de type acrylique. Par « polymère de type acrylique », on entend tout polymère dérivant de l'acide acrylique et de ses dérivés comme par exemple l'acrylate, les esters tel que l'acrylate de méthyle et l'acrylonitrile.

L'invention a donc pour objet un réseau tridimensionnel de fibres de polymère de type acrylique réticulé caractérisé en ce que :
- le diamètre des dites fibres est compris entre 0,1 et 1,5 µm, de préférence entre 0,3 et 1 µm, et plus préférentiellement entre 0,6 et 0,8 µm,
- la taille des interstices entre lesdites fibres est comprise entre 0,1 et 50 µm², de préférence entre 0,5 et 10 µm², et plus préférentiellement entre 1 et 2 µm², et
- la rigidité dudit réseau est caractérisée par un module d'élasticité compris entre 0,01 et 10 000 kPa, de préférence entre 0,1 et 10 000 kPa, de préférence entre 0,1 et 1 000 kPa, plus préférentiellement entre 0,1 et 300 kPa.

Selon un exemple particulier, ledit polymère plastique comprend au moins 50% de PAN, le pourcentage de PAN étant exprimé en pourcentage de la masse totale des différents polymères.

Selon cet aspect particulier, ledit réseau 3D est constitué de fibres dont le cœur est constitué de tout matériau approprié tel que notamment un polymère, ou un gel.

Selon un aspect plus particulier d'un réseau 3D selon l'invention, lesdites fibres sont entièrement constituées d'un polymère réticulé. Selon cet aspect, un réseau 3D selon l'invention est constitué de fibres comprenant au moins 10 %, au moins 20 %, au moins 30 %, au moins 40 %, au moins 50 %, au moins 60 %, au moins 70 %, au moins 80 % ou au moins 90 % d'un polymère réticulé, ce pourcentage étant exprimé en pourcentage massique desdites fibres.

Selon un autre mode de réalisation particulier, un réseau 3D selon l'invention est constitué de fibres de polymère réticulé, ledit polymère réticulé comprenant au moins un composé rigidifiant, ledit composé rigidifiant étant de préférence choisi parmi les nanocharges, et plus préférentiellement parmi : les nanoparticules, les nanotubes, les nanofibres et les nanofeuillets.

Selon un mode de réalisation particulier, un réseau selon l'invention est constitué de fibres comprenant au moins 10 %, au moins 20 %, au moins 30 %, au moins 40 %, au moins 50 %, au moins 60 %, au moins 70 %, au moins 80 % ou au moins 90 % d'un polymère réticulé dérivé de PAN, ce pourcentage étant exprimé en pourcentage massique de la solution de PAN avant sa réticulation.

Selon un aspect particulier, le diamètre des fibres d'un réseau 3D selon l'invention est compris entre 0,1 et 1,5 µm, de préférence entre 0,2 et 1,2 µm, de préférence entre 0,3 et 1,1 µm, de préférence entre 0,4 et 1 µm, de préférence entre 0,5 et 0,9 µm, et plus préférentiellement entre 0,6 et 0,8 µm, cette valeur représentant la moyenne des diamètres des fibres mesurés au sein dudit réseau. Ledit diamètre est mesuré par tout moyen connu par un homme du métier, et notamment par microscopie électronique à balayage.

Par «interstice entre lesdites fibres» on entend l'espace libre, ou « pore », ou « maillage » situé entre au moins deux fibres du réseau, dans lequel au moins une cellule, lorsqu'elle est mise en contact avec ledit réseau, est susceptible de se placer. Dans un réseau 3D selon l'invention, avant toute éventuelle fonctionnalisation de la surface des fibres, la taille des interstices est la dimension moyenne de la surface desdits interstices, cette taille est comprise entre 0,1 et 50 µm², de préférence entre 1 et 10 µm², et plus préférentiellement entre 1 et 2 µm². Cette dimension est mesurée notamment par microscopie électronique ou microscopie optique confocale. Selon une autre définition, la porosité d'un réseau selon l'invention est définie par un seuil de filtration compris entre 0,1 et 50 µm, de préférence entre 1 et 10 µm, et plus préférentiellement entre 1 et 2 µm. Cette dimension est définie avant toute éventuelle fonctionnalisation de la surface des fibres comme par exemple leur recouvrement par une protéine de la matrice extracellulaire, ladite fonctionnalisation ne modifiant pas sensiblement la taille des interstices entre les fibres.

Par « rigidité » on entend une caractéristique définie par le module d'élasticité. La rigidité d'un réseau selon l'invention est caractérisée par un module d'élasticité compris entre 0,01 et 10 000 kPa, de préférence entre 0,1 et 10 000 kPa, de préférence entre 0,1 et 1 000 kPa, de préférence entre 0,1 kPa et 300 kPa. Le module d'élasticité est mesuré par tout moyen connu de l'homme du métier et notamment par microscopie à force atomique, mettant en œuvre l'application d'une force variant du picoNewton au nanoNewton. De préférence, et sauf indication contraire, la rigidité est mesurée en tant que valeur moyenne sur l'ensemble de l'image enregistrée. Alternativement, la rigidité peut être mesurée au regard des fibres elles-mêmes, en excluant les interstices entre les fibres.

Selon un aspect particulier, un réseau 3D selon l'invention est caractérisé par des fibres alignées entre elles, ou suivant une orientation générale sans toutefois être nécessairement strictement parallèles. Selon un autre aspect particulier, un réseau 3D selon l'invention est caractérisé par des fibres organisées de façon aléatoire.

Selon un aspect plus particulier, l'invention a pour objet un réseau 3D de fibres de polymère réticulé obtenu par la réticulation thermique d'une solution de polymère de type acrylique. Selon un aspect plus particulier de l'invention, ladite solution comprend un polymère de type acrylique à l'exclusion de tout autre polymère. Selon un autre aspect plus particulier, ladite solution comprend un polymère de type acrylique et un second polymère. Selon un aspect encore plus particulier de l'invention, ladite solution comprend un premier polymère de type acrylique et un second polymère de type acrylique.

Selon un aspect plus particulier, l'invention a pour objet un réseau 3D de fibres de polymère réticulé obtenu par la réticulation thermique d'une solution de polyacrylonitrile (PAN). Selon un aspect plus particulier de l'invention, ladite solution comprend du PAN à l'exclusion de tout autre polymère, selon un autre aspect plus particulier, ladite solution comprend du PAN sous forme de copolymère.

Par « polyacrylonitrile » on entend un composé de formule (C₃H₃N)n de formule (I) suivante :

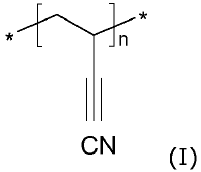

Selon un aspect plus particulier, un réseau 3D selon l'invention est constitué de fibres dont la surface comprend au moins 10 %, au moins 20 %, au moins 30 %, au moins 40 %, au moins 50 %, au moins 60 %, au moins 70 %, au moins 80 % au moins 90 %, au moins 95 % ou au moins 98 % d'un polymère réticulé obtenu par la réticulation thermique de PAN, ledit pourcentage étant exprimé en pourcentage massique de la surface desdites fibres.

Selon un aspect encore plus particulier, un réseau 3D selon l'invention est constitué de fibres comprenant au moins 10 %, de préférence au moins 20 %, au moins 30 %, au moins 40 %, au moins 50 %, au moins 60 %, au moins 70 %, au moins 80 % au moins 90 %, au moins 95 % ou au moins 98% d'un polymère réticulé obtenu par la réticulation thermique de PAN, ce pourcentage étant exprimé en pourcentage massique desdites fibres.

Selon un autre aspect particulier, un réseau 3D selon l'invention présente des propriétés de fluorescence, en particulier aux longueurs d'ondes suivantes : en vert (488 nm) et rouge (594 nm), et dans une moindre mesure en bleu (350 nm) et dans l'infrarouge (647 nm). Ces propriétés sont mesurables notamment par microscopie de fluorescence, par excitation à 488, 594, 350 et/ou 647 nm. Ces propriétés sont également mesurables par la technique dite de Lambda Scan, par exemple en appliquant au matériau une excitation aux longueurs d'onde d'excitation 405, 458, 488, 524, 561 et/ou 633 nm puis en déterminant la longueur d'onde et l'intensité du signal émis.

Selon un aspect particulier, l'invention a pour objet un réseau 3D de polymère réticulé caractérisé en ce que :
- ledit polymère réticulé est obtenu par la réticulation thermique d'une solution de polyacrylonitrile,
- le diamètre desdites fibres est compris entre 0,1 et 1,5 µm, de préférence entre 0,3 et 1 µm, et plus préférentiellement entre 0,6 et 0,8 µm,
- la taille des interstices entre lesdites fibres est comprise entre 0,1 et 50 µm², de préférence entre 0,5 et 10 µm², et plus préférentiellement entre 1 et 2 µm², et
- la rigidité dudit réseau est caractérisée par un module d'élasticité compris entre 0,1 et 10 000 kPa, de préférence entre 0,1 kPa et 300 kPa,
- optionnellement, la surface des fibres est recouverte par au moins une protéine de la matrice extracellulaire. Parmi ces protéines, on peut citer notamment la laminine, la fibronectine, la vitronectine, l'acide hyaluronique et le collagène.

Selon un aspect encore plus particulier, l'invention a pour objet un réseau 3D de polymère réticulé caractérisé en ce que :
- ledit polymère réticulé est obtenu par la réticulation thermique d'une solution de polyacrylonitrile,
- le diamètre desdites fibres est compris entre 0,6 et 0,8 µm,
- la taille des interstices entre lesdites fibres est comprise entre 1 et 2 µm², et
- la rigidité dudit réseau est caractérisée par un module d'élasticité compris entre 0,1 et 10 000 kPa, de préférence entre 0,1 et 300 kPa,
- optionnellement, la surface des fibres est recouverte par au moins une protéine de la matrice extracellulaire, notamment la laminine.

Selon cet aspect encore plus particulier, ledit réseau 3D de fibres est adapté en tant que support de cellules de glioblastome.

Selon un aspect encore plus particulier, l'invention a pour objet un réseau 3D de fibres de polymère réticulé, caractérisé en ce que ledit polymère comprend un composé rigidifiant. Un tel composé rigidifiant est de préférence choisi parmi les composés bien connus à cet effet par un homme du métier. Il peut notamment être choisi parmi les nanocharges, et de préférence parmi : les nanoparticules, les nanotubes, les nanofibres et les nanofeuillets. Plus préférentiellement, ledit composé rigidifiant est choisi parmi les nanotubes de carbone, notamment par les nanotubes de carbone multifeuillets (Multi Wall Carbon tubes ou MWCT) qui peuvent être facilement dispersés par l'homme de l'art dans un solvant adapté au polymère, lesdits nanotubes pouvant être fonctionnalisés en surface par des fonctions -COOH ou -NH₂ pour favoriser leur dispersion. Selon cet aspect, ledit composé rigidifiant est présent au sein dudit polymère réticulé, il n'est donc pas additionné au réseau 3D après la synthèse de celui-ci mais au contraire il est incorporé au polymère.

Selon un aspect particulier, au moins l'enveloppe desdites fibres du réseau comprend ledit polymère réticulé incorporant un composé rigidifiant, à une concentration de composé rigidifiant comprise entre 0,00001 et 5 %, de préférence entre 0,00001 et 1 %, de préférence entre 0,0001 et 0,1 %, de préférence entre 0,0002 et 0,08 %, de préférence entre 0,00075 et 0,05 %, exprimé en pourcentage massique de la solution dudit polymère avant réticulation.

Selon un aspect plus particulier, ledit composé rigidifiant est présent au sein du matériau constituant l'enveloppe et le cœur de ladite fibre.

Selon un aspect encore plus particulier, l'invention a pour objet un réseau 3D de fibres de polymère réticulé caractérisé en ce que la surface desdites fibres est recouverte par au moins une protéine de la matrice extracellulaire. Parmi les protéines de la matrice extracellulaire, on peut citer notamment la laminine, la fibronectine, la vitronectine, l'acide hyaluronique et le collagène.

Par « fibre recouverte par au moins une protéine» on entend une fibre à la surface de laquelle est présente une protéine, à une densité de protéines supérieure ou égale à 10 µg/cm². Comme observé par microscopie, lesdites protéines peuvent être présentes de manière discontinue à la surface desdites fibres.

Selon un aspect particulier, un réseau 3D de fibres de polymère réticulé selon la présente invention est caractérisé en ce que la surface des fibres du réseau est recouverte par au moins une protéine du milieu extracellulaire, de préférence la laminine.

Selon un aspect particulier, un réseau 3D de fibres de polymère réticulé selon la présente invention est caractérisé en ce que :
- ledit polymère réticulé est obtenu par la réticulation thermique de PAN,
- le diamètre des fibres est compris entre 0,1 et 1,5 µm, de préférence entre 0,3 et 1 µm, et plus préférentiellement entre 0,6 et 0,8 µm,
- la taille moyenne des interstices entre les fibres est comprise entre 0,1 et 50 µm², de préférence entre 0,5 et 10 µm², plus préférentiellement entre 1 et 2 µm²,
- la rigidité dudit réseau est caractérisée par un module d'élasticité compris entre 0,01 et 10 000 kPa, de préférence entre 0,1 et 10 000 kPa, de préférence entre 0,01 kPa et 1 000 kPa, de préférence entre 0,1 et 300 kPa,
- la taille des interstices entre les fibres est comprise entre 0,1 et 50 µm², de préférence entre 0,5 et 10 µm², plus préférentiellement entre 1 et 2 µm²,
- le polymère réticulé comprend un composé rigidifiant, choisi de préférence parmi les nanotubes de carbone, à une concentration comprise entre 0,00001 et 5 %, de préférence entre 0,0001 et 1 %, de préférence entre 0,0001 et 0,1 %, de préférence entre 0,0002 et 0,08°%, de préférence entre 0,00075 et 0,05 %, exprimée en pourcentage massique desdites fibres,
- le réseau émet une lumière fluorescente lorsqu'il est soumis à une énergie lumineuse, et
- la surface des fibres du réseau est recouverte par au moins une protéine du milieu extracellulaire, de préférence la laminine.

Selon un aspect encore plus particulier, l'invention a pour objet un réseau 3D de fibres de PAN, caractérisé en ce que :
- le diamètre des fibres est compris entre 0,3 et 1 µm,
- les fibres comprennent des nanotubes de carbone à une concentration comprise entre 0,0002 et 0,08°%, de préférence comprise 0,00075 et 0,05 %, exprimée en pourcentage massique desdites fibres,
- la rigidité du réseau est caractérisée par un module d'élasticité compris entre 0,1 et 1 000 kPa, de préférence entre 0,1 et 300 kPa, et
- la surface des fibres est recouverte de laminine.

L'invention a pour deuxième objet un procédé de préparation d'un réseau 3D de fibres selon l'invention, le procédé comprenant les étapes suivantes :
a) une étape de synthèse dudit réseau par électrofilage d'une solution de polymère de type acrylique dont la concentration est comprise entre 5 et 25%, de préférence entre 8 et 12%, de préférence 10 %, exprimée en pourcentage massique de ladite solution de polymère, pour obtenir un réseau 3D de fibres, et
b) une étape de traitement thermique sous atmosphère oxydante et à une température comprise entre 40 °C et 400 °C, de préférence entre 200°C et 300°C, de préférence entre 100 °C et 300 °C, du réseau 3D de fibres obtenu lors de l'étape a),
ladite étape a) de synthèse par électrofilage étant caractérisée en ce que :
- la solution de polymère est projetée par une aiguille dont l'amplitude de déplacement est comprise entre 30 et 50 mm, de préférence 40 mm, et dont la vitesse de déplacement est comprise entre 2 et 10 mm/seconde, de préférence de 5 mm/seconde,
- la distance entre la source de polymère et l'électrode collectrice est comprise entre 1 et 50 cm, de préférence entre 10 et 30 cm, plus préférentiellement de 15 cm, et le champ électrique appliqué dans le champ de projection est compris entre 16 et 24 kV, de préférence de 20 kV, et
- le débit de la solution de polymère pour l'alimentation de la seringue est compris entre 0,5 et 8,6 mL/h.

Plus particulièrement, dans un procédé de préparation d'un réseau 3D de fibres selon l'invention, ladite solution de polymère de type acrylique comprend du polyacrylonitrile (PAN) et un autre polymère de type acrylique, la concentration totale en polymère étant comprise entre 8 et 12%, de préférence 10 %.

Plus particulièrement, l'invention a pour deuxième objet un procédé de préparation d'un réseau 3D de fibres selon l'invention, le procédé comprenant les étapes suivantes :
a) une étape de synthèse dudit réseau par électrofilage d'une solution de PAN dont la concentration est comprise entre 8 et 12%, de préférence 10 %, exprimée en pourcentage massique de ladite solution de PAN, pour obtenir un réseau 3D de fibres, et
b) une étape de traitement thermique sous atmosphère oxydante et à une température comprise entre 40 °C et 400 °C, de préférence entre 200 °C et 300 °C, du réseau 3D de fibres obtenu lors de l'étape a),
ladite étape a) de synthèse par électrofilage étant caractérisée en ce que :
- la solution de PAN est projetée par une aiguille dont l'amplitude de déplacement est comprise entre 30 et 50 mm, de préférence 40 mm, et dont la vitesse de déplacement est comprise entre 2 et 10 mm/seconde, de préférence de 5 mm/seconde,
- la distance entre la source de polymère et l'électrode collectrice est comprise entre 1 et 50 cm, de préférence entre 10 et 30 cm, plus préférentiellement de 15 cm, et le champ électrique appliqué dans le champ de projection est compris entre 16 et 24 kV, de préférence de 20 kV, et
- le débit de la solution de polymère pour l'alimentation de la seringue est compris entre 0,5 et 8,6 mL/h.

Selon cet aspect, ladite solution est préparée à partir de PAN sont le degré de pureté est supérieur ou égale à 90 %, de préférence supérieure ou égale à 92 %, de préférence supérieure ou égale à 95 %, 96 %, 97 %, 98 % ou 99 %. Selon un autre aspect, ledit PAN est caractérisé par une masse molaire moyenne en masse (Molecular Weight ou MW) de 150 000. Le PAN est commercialisé par différents fournisseurs connus. Le PAN commercialisé par la société Sigma-Aldrich^{®} est particulièrement adapté pour cet aspect de l'invention.

Selon un premier aspect particulier, un procédé selon l'invention est caractérisé en ce que la solution de PAN est projetée sur une électrode collectrice rotative, de préférence un tambour, de diamètre compris entre 12 et 18 cm, de préférence de 15 cm, la vitesse de rotation de ladite électrode étant comprise entre 1 et 100 000 g, de préférence entre 1 et 1 000 g et plus préférentiellement de 335 g. La mise en œuvre d'un tel procédé conduit à l'obtention d'un réseau comprenant des fibres alignées, ledit réseau de fibres alignées présentant une orientation générale plutôt que des fibres strictement parallèles. Selon un autre aspect particulier, dans un procédé selon l'invention la solution de PAN est projetée sur un collecteur plan non rotatif, la mise en œuvre de cet autre aspect du procédé conduit à synthèse d'un réseau 3D comprenant des fibres enchevêtrées, dans lequel les fibres ne présentent aucune orientation générale.

Selon un autre aspect particulier, un procédé selon l'invention est caractérisé en ce que la solution de PAN ne comprend pas de composé rigidifiant, tel que des nanoparticules, des nanotubes, des nanofibres ou des nanofeuillets. Plus particulièrement, un procédé selon l'invention met en œuvre une solution de PAN comprenant des nanotubes de carbone, et notamment des nanotubes de carbone multifeuillets (MWCT). Selon cet aspect particulier, l'étape de synthèse par électrofilage est caractérisée en ce que :
- le champ électrique appliqué dans le champ de projection est compris entre 16 et 24 kV, de préférence de 20 kV, et
- le débit de la solution de polymère pour l'alimentation de la seringue est compris entre 0,5 et 8,6 mL/h, de préférence entre 2,1 et 2,7 mL/h, et de préférence 2,4 mL/h.

Selon un autre aspect particulier, un procédé selon l'invention est caractérisé en ce que la solution de PAN comprend un composé rigidifiant, de préférence choisi parmi les nanocharges, et plus préférentiellement parmi : les nanoparticules, les nanotubes, les nanofibres et les nanofeuillets. Plus particulièrement, un procédé selon l'invention met en œuvre une solution de PAN comprenant des nanotubes de carbone, et notamment des nanotubes de carbone multifeuillets (MWCT).

Selon un aspect plus particulier, les fibres dudit réseau comprennent un composé rigidifiant, à une concentration comprise entre 0,00001 et 5 %, de préférence entre 0,00001 et 1 %, de préférence entre 0,0001 et 0,1 %, de préférence entre 0,002 et 0,08 %, et de préférence entre 0,0075 et 0,05 %, exprimé en pourcentage massique de la solution du polymère avant réticulation.

Selon un autre aspect particulier, un procédé selon l'invention comprend une étape d'électrofilage d'une solution d'un polymère, ladite solution comprenant un composé rigidifiant, de préférence constitué par des nanotubes de carbone, et un agent dispersant, de préférence un tensioactif et plus préférentiellement un tensioactif non ionique. La présence dudit agent dispersant assure une bonne solubilisation dudit agent rigidifiant.

Selon cet aspect particulier, un procédé selon l'invention est caractérisé en ce que la solution de PAN comprend un composé rigidifiant, choisi parmi les nanoparticules, les nanotubes, les nanofibres et les nanofeuillets, de préférence les nanotubes de carbone, et notamment des nanotubes de carbone multifeuillets (MWCT), et l'étape de synthèse par électrofilage est caractérisée en ce que :
- le champ électrique appliqué dans le champ de projection est compris entre 16 et 24 kV, de préférence de 20 kV, et
- le débit de la solution de polymère pour l'alimentation de la seringue est compris entre 0,5 et 8,6 mL/h, de préférence entre 0,5 et 1,9, mL/h et de préférence entre 0,8 et 1,5 mL/h.

Plus particulièrement, un procédé selon l'invention est caractérisé en ce que l'étape b) de traitement thermique appliqué au réseau 3D comprend le maintien sous atmosphère oxydante dudit réseau pendant au moins 10 minutes, de préférence entre 10 et 300 minutes, de préférence entre 20 et 180 minutes, entre 60 et 150 minutes, plus préférentiellement pendant 120 minutes, à une température comprise entre 40 et 400 °C, de préférence entre 100 et 300 °C, plus préférentiellement à une température de 250 °C. Ladite étape de traitement thermique sous atmosphère oxydante est précédée par une montée en température et suivie par une descente en température, selon les caractéristiques du dispositif utilisé pour l'application dudit traitement thermique.

Lors dudit traitement thermique, la couleur du réseau passe de blanc à marron, traduisant l'aromatisation des fibres et la réticulation du polymère.

Plus particulièrement selon ce deuxième aspect, l'invention a pour objet un procédé comprenant les étapes suivantes :
a) une étape de synthèse dudit réseau par électrofilage d'une solution de 10 % de PAN comprenant 0,05 % de nanotubes de carbone MWCT, ladite étape a) étant caractérisée en ce que :
   - la solution de PAN est projetée par une aiguille dont l'amplitude de déplacement est comprise entre 30 et 50 mm et dont la vitesse de déplacement est comprise entre 2 et 10 mm/seconde,
   - la distance entre la source de polymère et l'électrode collectrice est comprise entre 1 et 50 cm, et le champ électrique appliqué dans le champ de projection est de 20 kV, et
   - le débit de la solution de polymère pour l'alimentation de la seringue est de 2,4 ml/h.
b) une étape de traitement thermique sous atmosphère oxydante et à une température comprise entre 200 °C et 300 °C, pendant 120 minutes, du réseau 3D de fibres obtenu lors de l'étape a).

Selon un autre aspect particulier, un procédé selon l'invention est caractérisé en ce que ladite étape de traitement thermique est suivie par le traitement de la surface des fibres du réseau, destiné à fonctionnaliser lesdites fibres, et notamment contribuer à mimer l'environnement extracellulaire des cellules en contact avec un réseau 3D selon l'invention. Selon un aspect particulier d'un procédé selon l'invention, l'étape de traitement de surface comprend la mise en présence du réseau avec une solution d'au moins une protéine de la matrice extracellulaire, notamment choisie parmi : la laminine, la fibronectine, la vitronectine, l'acide hyaluronique et le collagène. Cette solution d'au moins une protéine de la matrice extracellulaire est préparée dans un tampon approprié et dans des conditions de concentration appropriées. Par exemple, la laminine est préparée dans du tampon phosphate salin (PBS) à une concentration comprise entre 1 et 2 mg/ml, le réseau est ensuite mis en contact avec une solution de laminine de concentration comprise entre 5 et 20 µg/ml.

Dans un procédé selon l'invention, la mise en présence du réseau avec une solution d'au moins une protéine de la matrice extracellulaire est optionnellement précédée par un traitement préalable de la surface des fibres dudit réseau, destiné à améliorer la qualité de la fonctionnalisation dudit réseau. Ledit traitement préalable est mis en œuvre par tout procédé adapté, connu de l'homme du métier, notamment par un dépôt chimique, mis en œuvre en phase liquide ou gazeuse. Selon un aspect préféré, un tel dépôt chimique comprend la mise en présence, notamment par immersion, avec une solution de poly-D-lysine à une concentration comprise entre 5 et 50 µg/ml.

Selon cet aspect particulier, un procédé selon l'invention comprend le traitement de la surface des fibres du réseau 3D au moyen des étapes successives suivantes :
- traitement préalable de la surface des fibres,
- traitement de la surface conduisant à la fonctionnalisation des fibres avec au moins une protéine de la matrice extracellulaire choisie parmi : la fibronectine, la vitronectine, l'acide hyaluronique et le collagène, et de préférence la laminine.

Selon un aspect plus particulier, un procédé selon l'invention comprend le traitement de la surface des fibres du réseau 3D au moyen des étapes successives suivantes :
- traitement préalable avec une solution de poly-D-lysine,
- traitement de la surface avec une solution de laminine.

L'invention a pour troisième objet un dispositif pour le support de cellules, comprenant un réseau 3D infusible de fibres de polymère réticulé selon l'invention ou un réseau obtenu par un procédé selon l'invention.

Un dispositif pour le support de cellules selon l'invention comprend un réseau 3D infusible de fibres de polymère réticulé selon l'invention, plus particulièrement un réseau 3D infusible de fibres de polymère de type acrylique réticulé selon l'invention ou un réseau obtenu par un procédé selon l'invention, en association avec tout autre élément.

Un tel dispositif peut notamment être constitué par ledit réseau en tant que tel, ou par ledit réseau disposé ou inséré dans tout support approprié, comme par exemple une boite de culture. A titre d'exemple, un dispositif selon l'invention peut être constitué par une plaque de type plaque multi-puits dont au moins un puits comprend un réseau 3D de fibres selon l'invention. Selon un aspect particulier, l'invention a pour objet un dispositif comprenant un ou plusieurs réseaux 3D selon l'invention, identiques ou différents, disposés ou insérés au sein de tout support approprié de type « plaque multi puits ».

L'invention a pour quatrième objet l'utilisation en tant que support de cellules d'un réseau 3D selon l'invention, d'un réseau 3D obtenu par un procédé selon l'invention ou d'un dispositif comprenant un tel réseau 3D. Une telle utilisation comprend successivement :
- la mise en contact de cellules d'intérêt avec un réseau 3D selon l'invention, d'un réseau 3D obtenu par un procédé selon l'invention ou d'un dispositif comprenant un tel réseau 3D, dans des conditions et pendant une durée appropriées, et
- l'observation et éventuellement la détermination d'au moins un paramètre caractérisant lesdites cellules et/ou l'analyse biochimique desdites cellules.

Lesdites conditions et durée appropriée sont bien connues de l'homme du métier spécialiste de la culture cellulaire, et sont notamment relatives au milieu dans lequel sont suspendues les cellules, leur concentration et leur température d'incubation.

Les paramètres caractérisant les cellules sont notamment choisis parmi : l'état de vie ou de mort cellulaire, le nombre de cellule, la localisation des cellules à la surface et/ou au sein dudit réseau, la présence de cellules isolées et/ou groupées, la taille et la forme des cellules, la caractérisation du noyau et des organites. Les moyens d'observation desdites cellules sont notamment, mais non exclusivement, tout type de microscopie, comme par exemple électronique à balayage, la microscopie à fluorescence classique, la microscopie confocale, la microscopie multiphoton, la microscopie à épifluorescence, la vidéo microscopie et l'imagerie tridimensionnelle.

L'analyse biochimique des cellules comprend notamment, mais non exclusivement : l'analyse qualitative et/ou quantitative de l'ADN, de l'ARN, des protéines cellulaires et des métabolites, par tout moyen approprié connu par un homme du métier. De tels moyens incluent notamment les études transcriptomiques et protéomiques.

Selon un aspect plus particulier, l'invention a pour objet l'utilisation d'un réseau 3D selon l'invention, d'un réseau 3D obtenu par un procédé selon l'invention ou d'un dispositif comprenant un tel réseau 3D en tant que support de cellules prolifératives ou cancéreuses. Selon un aspect encore plus particulier, l'invention a pour objet l'utilisation d'un réseau 3D selon l'invention, d'un réseau 3D obtenu par un procédé selon l'invention ou d'un dispositif comprenant un tel réseau 3D en tant que support de cellules de glioblastome, plus particulièrement de cellules invasives de glioblastome (Glioblastome Invasive Cells).

Selon un autre aspect plus particulier, l'invention a pour objet l'utilisation d'un réseau 3D selon l'invention, d'un réseau 3D obtenu par un procédé selon l'invention ou d'un dispositif comprenant un tel réseau 3D pour l'étude et la caractérisation de la survie, de la prolifération, de la différentiation et/ou de la migration de cellules. Selon cet aspect, lesdites cellules sont notamment des cellules cancéreuses, et plus particulièrement des cellules de glioblastome.

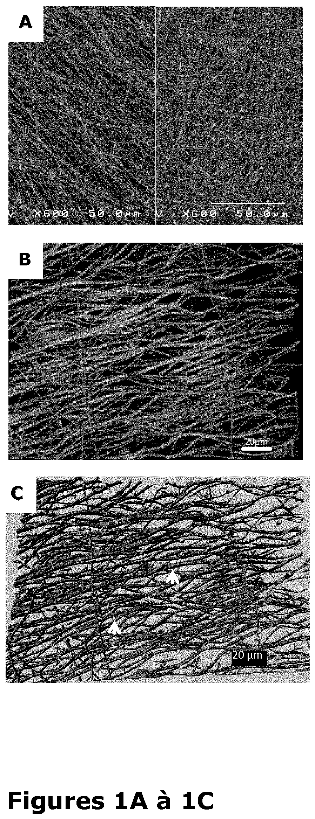

Les exemples 1 à 4 et les figures 1 à 10 qui suivent illustrent l'invention.

### DESCRIPTION DES FIGURES

Les figures 1A à 1C illustrent la caractérisation physique d'un réseau 3D de fibres selon l'invention, conformément à l'exemple 1 : Fig. 1A : images de microscopie électronique à balayage (barre d'échelle : 50 µm) de fibres de PAN alignées (image de gauche) ou non alignées (image de droite) ; fig. 1B : image de l'immunocoloration montrant les dépôts de laminine sur les fibres (barre d'échelle : 20 µm), fig. 1C : reconstitution 3D à partir d'un « z-stack » correspondant à l'acquisition de l'image de la fig. 1B, les dépôts discontinus de laminine sur les fibres sont indiqués par des flèches (barre d'échelle : 20 µm).

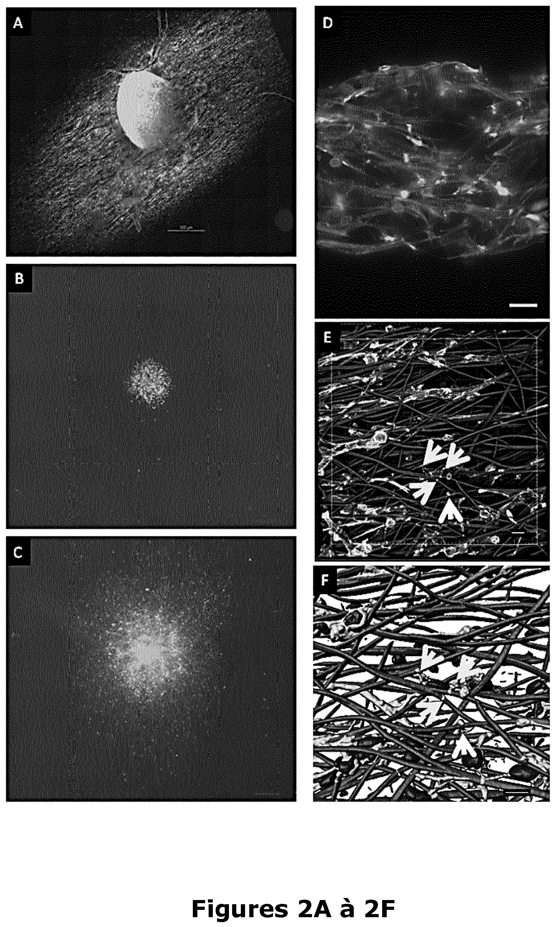

Les figures 2A à 2F illustrent l'adhérence et la pénétration des neurosphères (NS) au sein du réseau de fibres, selon l'exemple 2 : Fig. 2A : image montrant l'adhérence et la pénétration des neurosphères dans le réseau 3D (barre d'échelle : 500 µm), au moment du dépôt une neurosphère contient 5.000 cellules ; fig. 2B : image montrant les neurosphères de cellules Gli4 5 heures après dépôt sur le réseau de fibres (barre d'échelle : 200 µm) ; fig. 2C : image montrant les neurosphères de cellules Gli4 6 jours après dépôt sur le réseau de fibres (barre d'échelle : 200 µm), fig. 2D : vue latérale de la répartition des cellules Gli4 migratoires, en s'éloignant des neurosphères et en profondeur dans le réseau de fibres (barre d'échelle : 20 µm), fig. 2E : image de l'immunocoloration montrant l'adhésion des cellules Gli4 aux fibres (barre d'échelle : 10 µm) les flèches indiquent les cellules Gli4 ; fig. 2F : reconstitution 3D à partir d'un « z-stack » correspondant à l'acquisition de l'image des cellules Gli4 sur les fibres (fig. 2E).

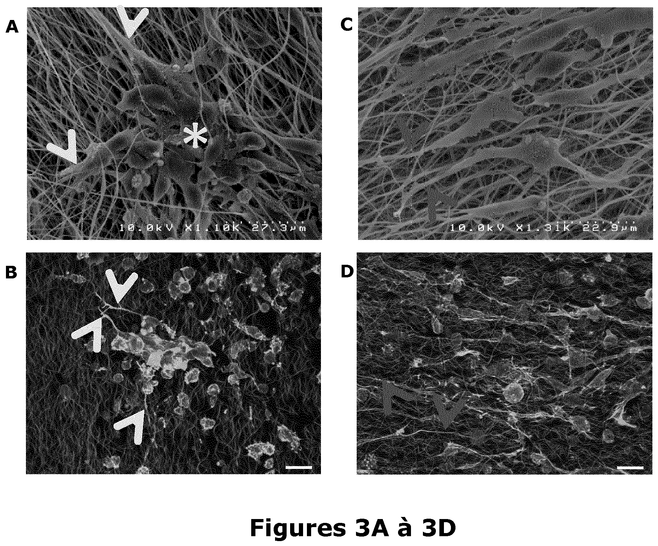

Les figures 3A à 3D illustrent la migration individuelle ou collective de cellules déposées sur un réseau 3D de fibres, selon l'exemple 2, après 6 jours de culture en conditions de différentiation, en absence (fig. 3A et 3B) ou en présence (fig. 3C et 3D) de laminine ; fig. 3A) et 3C : visualisation des cellules Gli4 par microscopie électronique à balayage (barre d'échelle : 500 µm), fig. 3B et 3D : visualisation des cellules Gli4 après marquage du cytosquelette d'actine à la phalloïdine verte (barre d'échelle : 20 µm). En fig. 3A et 3B, les flèches indiquent les extensions lamellipodes des cellules périphériques et les cellules rondes situées au centre d'un groupe de cellules migrant collectivement, en absence de laminine sur les fibres. En fig. 3C et 3D, les flèches indiquent les cellules migrant individuellement, en présence de laminine sur les fibres.

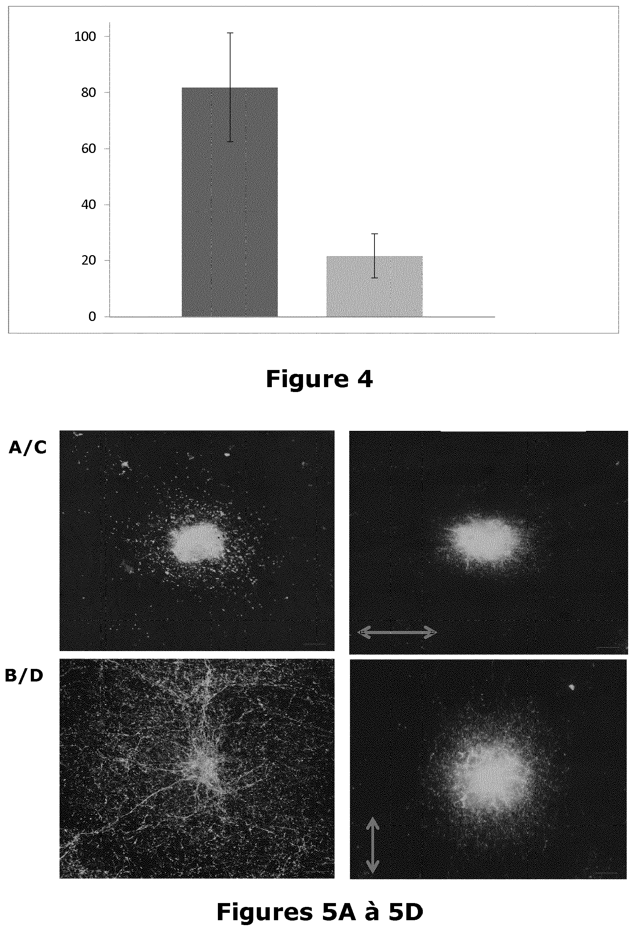

La figure 4 illustre le nombre de migrations collectives (en ordonnées) quantifié en présence (histogramme de gauche) ou en absence (histogramme de droite) de laminine sur les fibres alignées, les agrégats formés par au moins 2 cellules et séparés physiquement des neurosphères sont considérés comme une migration collective, les résultats sont représentatifs d'au moins 3 expériences différentes, ^{∗∗∗∗}p<0,0001 (test de Student).

Les figures 5A à 5D illustrent la direction de la migration des cellules sur des fibres alignées ou non-alignées, selon l'exemple 2, après 6 jours de culture en conditions de différentiation sur un réseau 3D de fibres : le squelette d'actine a été coloré par la phalloïdine et le noyau avec du colorant Hoechst 3342 (barre d'échelle 200 µm), la direction des fibres est indiquée par les flèches, fig. 5A et 5C : en absence de laminine, 5B et 5D : en présence de laminine, fig. 5A et 5B : fibres non-alignées, fig. 5C et 5D : fibres alignées.

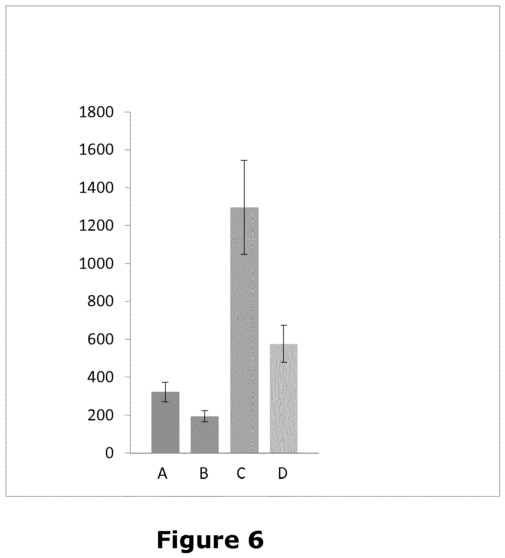

La figure 6 représente la quantification (en ordonnées) du nombre total de cellules Gli4 migratoires en direction parallèle (barres A et C) ou perpendiculaire (barres B et D) des fibres alignées, en absence (barres A et B) ou en présence (barres C et D) de laminine. Le nombre de cellules est compté à une distance comprise entre 200 µm et 2.000 µm du bord extérieur des neurosphères. Ce domaine a été choisi pour exclure la migration due à la croissance expansive des cellules près des neurosphères. Entre les barres A et B : ^{∗}p<0,1, entre les barres C et D : ^{∗∗}p<0,01 (test de Student). Tous les résultats sont représentatifs de deux expériences indépendantes.

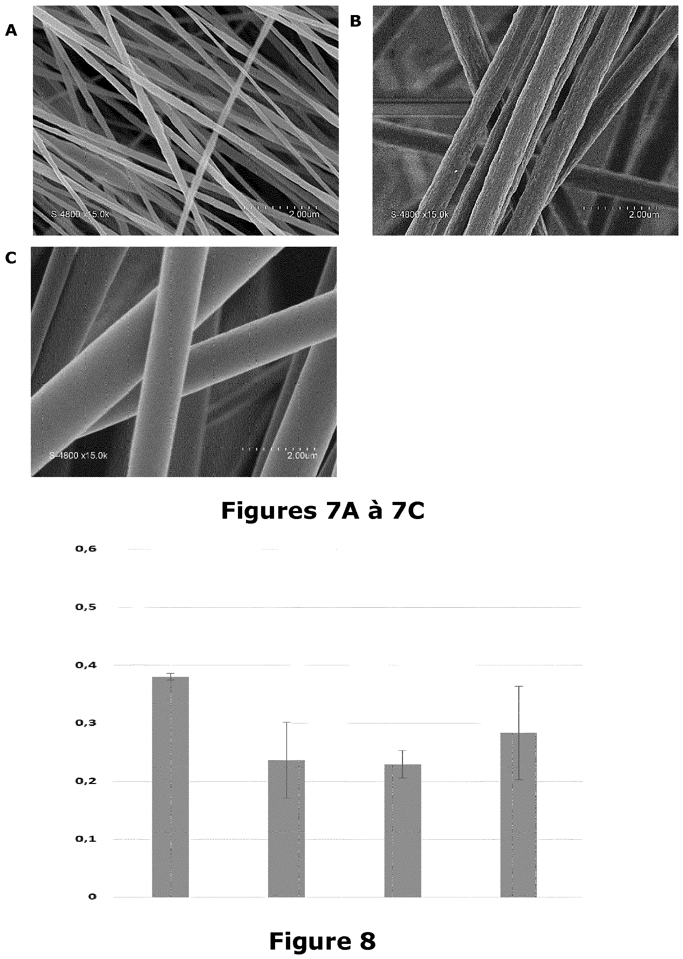

Les figures 7A à 7C représentent des images de microscopie électronique à balayage (barre d'échelle : 200 µm) de fibres de polymère réticulé, ledit polymère est obtenu par la réticulation de solutions de PMMA 5% + PAN 5% (Fig. 7A), PMMA 5% + PAN 10% (Fig. 7B) ou PMMA 20% (Fig. 7C).

La figure 8 (test MTT) est un histogramme illustrant en ordonnées l'absorbance du formazan produit par réduction du sel de tétrazolium MTT (bromure de 3-(4,5-dimethylthiazol-2-yl)-2,5-diphenyl tetrazolium) par les cellules vivantes et actives métaboliquement sur les fibres de polymère, en abscisses, de gauche à droite, PAN 10% ; PMMA 5% + PAN 5% ; PMMA 5%+ PAN 10% et PMMA 20%..

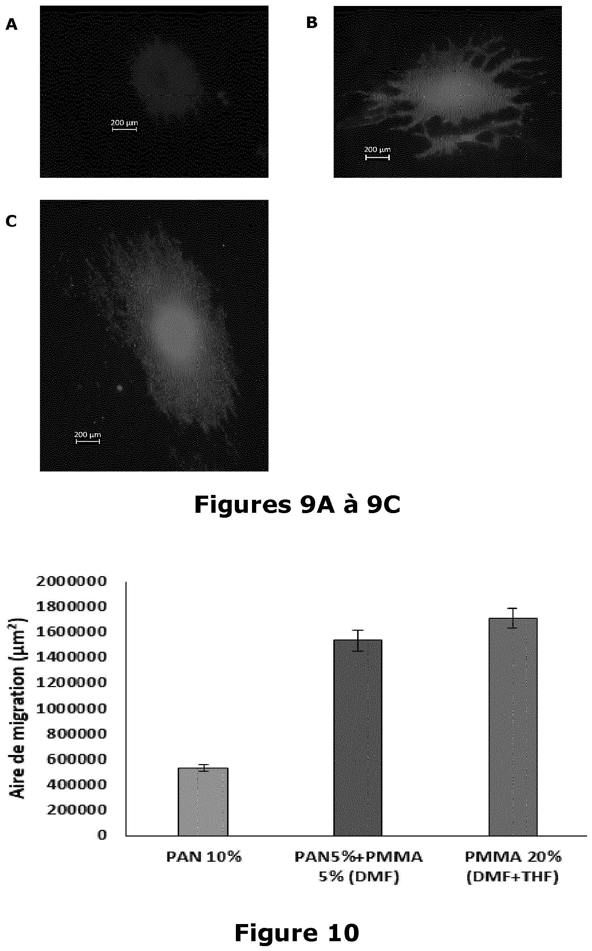

Les figures 9A à 9C représentent la direction de la migration de cellules Gli4 sur les fibres de PAN 10% (Fig. 9A), de PMMA 5% + PAN 5% (Fig. 9B), ou de PMMA 20% (Fig. 9C) après 5 jours de culture en conditions de différentiation sur un réseau 3D de fibres : le squelette d'actine a été coloré par la phalloïdine et le noyau avec du colorant Hoechst 3342.

La figure 10 représente un histogramme indiquant, en ordonnées, la surface de l'aire de migration (en µm²) des cellules Gli4 déposées sur les fibres de polymère, en abscisses, de gauche à droite, de PAN 10%, PAN5% + PMMA 5%, ou PMMA 20%.

### EXEMPLES

### EXEMPLE 1 : SYNTHESE D'UN RESEAU 3D DE FIBRES DE POLYACRYLONITRILE ET CARACTERISATION PHYSIQUE

### 1.1. Matériels et méthodes

### Synthèse du réseau 3D de fibres de PAN

Une solution à 10% massique de Polyacrylonitrile (Sigma Aldrich) est préparée dans du diméthylformamide. Pour 10 g de solution de PAN à 10 % : pesée à valeur d'1 g de PAN pour 9 g de DMF, puis chauffage à 75°C avec agitation jusqu'à dissolution complète du PAN dans le DMF.

Cette solution est mise en forme par électrofilage. Le collecteur est un collecteur rotatif de 15 cm de diamètre. Un champ électrique de 20 kV est appliqué entre l'aiguille distribuant le polymère et le collecteur.
- *Réseau 3D avec fibres alignées* : L'aiguille se trouve à une distance de 15 cm du collecteur rotatif. Le collecteur autour duquel s'enroulent les fibres possède une vitesse de rotation de 2.000 rpm permettant d'obtenir un réseau dont les fibres sont alignées.
- *Réseau 3D avec fibres dites "enchevêtrées" :* L'aiguille se trouve à une distance de 15 cm d'un collecteur plan, non rotatif.

Dans les deux cas, le polymère est acheminé jusqu'à l'aiguille grâce à un « pousse-seringue » avec une force de poussée de 2,4 ml/h dans le cas présent. Durant l'électrofilage, l'aiguille réalise des déplacements d'une amplitude de 40 mm avec une vitesse de déplacement de 5 mm/sec. L'électrofilage dure au moins 20 minutes. Le tissu de fibres subit ensuite un traitement thermique avant d'être stérilisé.

### Traitement thermique du réseau 3D

Le réseau obtenu par électrofilage est traité thermiquement sous air à une température de 250 °C, avec une vitesse de chauffe de 120 °C/heure, un palier à 250 °C pendant 2 heures, la vitesse de refroidissement est de 300 °C/h. Lors du traitement, la couleur du réseau passe de blanc à marron, traduisant l'aromatisation des fibres.

Avant utilisation, le réseau subit une stérilisation à l'éthanol 70° ou à l'autoclave.

### Synthèse d'un réseau 3D de fibres de PAN et de nanotubes de carbone

Lors de la réalisation des fibres contenant les nanotubes de carbones multiwall (MultiWallCarbonTubes) (Nanocyl^{™}, 95 % de pureté, produit référencé NC3101 ou NC3151) et pour augmenter la dispersion des MWCT dans la solution de PAN, le surfactant triton 100X est ajouté à 2,5 % de la solution finale pour 0,05 % WT de MWCT.

Pour une préparation d'une solution de PAN à 0,05 % WT, les étapes suivantes sont réalisées :
a) Préparation de 10 g d'une solution à 0,05 % (pourcentage massique) de MWCT dans du PAN 10 % :
   - Pesée à valeur d'1 g de PAN, 0,05 g de MWCT et 8,95 g de DMF,
   - Chauffage à 75°C avec agitation jusqu'à dissolution complète du PAN dans le DMF,
   - Sonication de la solution
b) Réalisation d'une solution de PAN à 10 % selon le paragraphe 1.1.1.
c) Mélange de 1 g de la solution de PAN 10 % + 0,05 % WT (pourcentage massique) de MWCT pour 0,75 g de Triton 100X et de 8,25 g de la solution de PAN à 10 %
d) Sonication de la solution mélangée.

La solution de polymère est ensuite traitée par électrofilage puis le réseau obtenu est traité thermiquement comme indiqué précédemment.

Par dilution avec du PAN 10%, un réseau constitué de fibres de PAN et de 0,0016 % (pourcentage massique) de MWCT est synthétisé

### Recouvrement de la surface des fibres par la laminine

Pour leur fonctionnalisation par la laminine, les plaques de culture et le réseau 3D de fibres électrofilées biocompatibles sont incubés pendant une nuit avec 25 µg/ml de poly-D-lysine en tampon borate à 37%, puis lavées 2 fois en eau stérile et incubées avec 5,2 µg/ml de laminine en eau stérile pendant 4 h à 37°. Deux rinçages à l'eau sont effectués.

### Inclusion du réseau de fibres dans des puits

Cette inclusion peut être réalisée grâce à leur découpage via un emporte-pièce de taille correspondant à la taille du puits destiné à accueillir les fibres. Les fibres découpées sont ensuite déposées au fond du puits et prêtes à l'utilisation.

Il est également possible de positionner les fibres découpées dans des inserts, de taille correspondant à la taille des puits, permettant de faciliter leur entrée et leur sortie des puits ou permettant la réalisation de gradients de concentration de part et d'autre des fibres.

### 1.2. Résultats

### Caractérisation d'un réseau 3D de fibres de PAN

Le diamètre des fibres, estimé à 0,68 +/- 0,28 µm, est similaire au diamètre des axones présents dans le corps calleux, estimé à 0,64 +/- 0,42 µm (Liewald et al., 2014, Biol. Cybern. 108, 541-557). Selon le procédé d'électrofilage mis en œuvre, le réseau de fibres généré comprend des fibres alignées ou organisées de façon aléatoire (fig. 1A). Le réseau de fibres alignées présente une orientation générale plutôt que des fibres strictement parallèles.

L'évaluation des propriétés mécaniques est réalisée au moyen de la microscopie à force atomique (Atomic Force Microscopy, en anglais). L'élasticité du réseau constitué de fibres de PAN non additionné de nanotubes de carbone est caractérisée par un module d'élasticité de 0,16 kPa. Les expériences sont conduites en mode contact sur un appareil Asylum MFP-3D (Asylum Research, Santa Barbara, CA, USA) monté sur un microscope inversé de marque Olympus, en utilisant un système levier-pointe triangulaire en nitrure de silicium (MLCTAUHW, Veeco) et avec une constante de raideur de 10 pN/nm. Les mesures sont conduites en appliquant une force maximale de 1 nN. La rigidité est mesurée en tant que valeur moyenne sur l'ensemble de l'image enregistrée.

Pour un réseau constitué de fibres de PAN et de 0,0016 % (pourcentage massique) de MWCT, l'élasticité est caractérisée par un module d'élasticité de 62 kPa.

Pour un réseau constitué de fibres de PAN et de 0,05 % (pourcentage massique) de MWCT, l'élasticité est caractérisée par un module d'élasticité de 250 kPa.

Les interstices sont tels que le maillage des fibres est permissif mais contraint néanmoins les cellules à se déformer pour entrer dans la matrice. Les interstices sont compris entre 0,5 µm² et 9 µm², ce qui correspond à une situation de confinement des cellules.

### Propriétés de fluorescence :

Lorsque le réseau est soumis à une énergie lumineuse, les fibres apparaissent comme fluorescentes (fig. 1B). Elles sont autofluorescentes en vert (488 nm) et rouge (594 nm) et, dans une bien moindre mesure, en bleu (350 nm) et dans l'infrarouge (647 nm). La fonctionnalisation des fibres avec de la laminine permet de modifier le microenvironnement.

### Caractérisation du réseau recouvert de laminine

Les dépôts de laminine sont distribués de façon discontinue sur les fibres (fig. 1C).

### Conclusion

Le réseau 3D ainsi obtenu est non cytotoxique, et peut constituer un support 3D pour les cellules, permettant leur prolifération, leur migration et leur différentiation.

### EXEMPLE 2 : CARACTERISATION DE LA MIGRATION DE CELLULES DE GLIOBLASTOME

### 2.1. Matériel et méthodes

### Culture de cellules de glioblastome

L'obtention et l'isolement de cellules de glioblastome (GBM) ainsi que les cultures sont réalisées en utilisant le protocole élaboré par Guichet *et al.* (Guichet et al, 2013, Glia, 61, 225-239), d'après Dromard *et al.* (Dromard et al., 2008, J. Neurosci. Res. 86, 1916-1926) pour les neurosphères non adhérentes. Les cellules Gli4 et GliT sont des cultures primaires de glioblastome dérivées de deux patients différents. Ces cellules de GBM sont cultivées dans deux conditions différentes en milieu DMEM/F12 supplémenté avec du glucose, de la glutamine, de l'insuline, du N2 et de la ciproflaxine.

En conditions non adhérentes, dites « de prolifération », les boîtes de culture sont prétraitées avec du poly-2 hydroxyéthyl méthacrylate (poly-HEME, SIGMA), le milieu est aussi supplémenté avec des facteurs de croissance EGF (epidermal growth factor) et FGF (Fibroblast Growth Factor), gentamycine, héparine, fungizone, fungine et B27. Dans ces conditions, les cellules de GBM forment des neurosphères (NS), réminiscentes de cellules souches neurales (NSC) in vitro, et expriment des progéniteurs nerveux et des marqueurs de cellules souches (nestine, olig2, sox2, etc...), s'auto-renouvellent et propagent des tumeurs chez des animaux immunocompromis. Après confluence, les NS sont dissociées mécaniquement en utilisant du HBSS (sans calcium ni magnésium) et re-ensemencées.

En conditions dites « de différentiation », le DMEM/F12 contient du sérum bovin fœtal (0,5 %) sans facteur de croissance et sans héparine. Dans ce cas, les cellules de GBM sont cultivées en adhérence sur une surface plane (2D) ou sur un réseau 3D préparé selon l'exemple 1, sans poly-HEME.

### Culture de cellules sur le réseau 3D

Avant le dépôt des cellules, les réseaux 3D de fibres electrofilées biocompatibles (désignées par : « fibres »), préparées comme décrit dans l'exemple 1, sont stérilisés en alcool à 70%, autoclavé et fonctionnalisés, ou non, avec de la poly-D-lysine-Laminine, comme décrit dans l'exemple 1. Pour la fonctionnalisation, les plaques pour la culture 2D et les fibres sont incubées 1 nuit avec 25 µg/ml de poly-D-lysine en tampon borate à 37%, puis lavées 2 fois en eau stérile et incubées avec 5,2 µg/ml de laminine en eau stérile pendant 4 h à 37°. Les plaques et les fibres sont lavés 2 fois en eau stérile et les neurosphères, ou les cellules dissociées, de Gli4 et GliT y sont ensemencées. Les cellules sont gardées en culture pendant 6 jours à 37° C et 5 % CO₂.

Pour obtenir des neurosphères, des microplaques Corning à 96 puits sont utilisées (Corning 7007). Les neurosphères dissociées de GBM sont cultivées en conditions de prolifération, et ensemencées à 5.000 cellules par puits. Les cellules sédimentent et les NS sont récoltées 24 heures après ensemencement.

### Xénotransplantation orthotopique de GBM

Les cellules Gli4 et GliT sont dissociées enzymatiquement en utilisant de la trypsine 0,25% et resuspendues dans du PBS à 0,5 x 10⁵ cellules par microlitre. 3 microlitres (1,5 x 10⁵ cellules) sont injectés dans le striatum (1 mm rostral, 2 mm latéral et 2,5 mm de profondeur) de souris MNRI-nude femelles âgées de 6 semaines (laboratoires Janvier) sous anesthésie à l'isoflurane. Une seringue Hamilton connectée à une pompe est utilisée pour injecter la suspension cellulaire avec un flux de 0,3 ml/min. A la fin de la chirurgie, les cellules restantes seront ensemencées pour vérifier la viabilité cellulaire. Après 3 mois, les animaux sont sacrifiés sous anesthésie au pentobarbital et fixés par une perfusion intracardiaque de PFA. Les cerveaux sont récupérés et post-fixés pendant une nuit en PFA 4 % puis immergés successivement en 7 %, 15 % et 30 % sucrase. Puis, ces cerveaux sont inclus en OCT (Optimal Cutting Temperature, température optimale de section), congelés en azote liquide et conservés à -20 °C. Des sections coronales de cerveaux de 14 µm d'épaisseur sont faites au cryostat.

### Cryosection des réseaux 3D

Les réseaux 3D de fibres sont inclus dans un composé OCT. L'épaisseur des sections latérales est de 14 µm.

### Immunofluorescence et reconstitution 3D

Après 6 jours de culture, les GSC cultivés en NF ou en plaques sont fixés avec du PFA 4%. Les réseaux 3D, les plaques et les sections de cerveaux sont bloqués et perméabilisés en utilisant du PBS-triton 0,5%- sérum de cheval 5%. Les anticorps primaires sont incubés sur la nuit à 4°C. Les anticorps secondaires couplés à un fluorochrome sont incubés 2 h à température ambiante à la dilution 1/500. Ces anticorps sont : N-cadherine (Abcam ab12221), calpaine-2 (Abcam ab155666) et noyau humain (human nuclei, Millipore MAB 1281). Les sections sont montées avec du fluoromount et séchées avant observation. Le cytosquelette actine est mis en évidence avec la phalloïdine (verte) et le noyau avec du Hoechst 33342. Les images sont prises avec une acquisition Z-stack en utilisant les microscopes Confocal 2 Zeiss LSM 5 Live DUO et Widefield 1-Zeiss Axioimager Z1/Zen (équipé avec un apotome). Le logiciel Imaris X64 8.1.2 est utilisé pour la reconstitution d'images 3D. Les quantifications sont réalisées avec le logiciel ZEN 2012.

Les cellules Gli4 cultivées sur les fibres sont fixées au glutaraldéhyde 2,5 % dans du tampon PHEM pendant une heure à température ambiante puis toute la nuit à 4°C. Les cellules sont ensuite déshydratées avec de l'alcool à 70 %, 96 % et 100 % successivement, puis incubées dans du HMDS pour séchage.

### Western blot

Les protéines sont extraites à partir des cultures sur fibres en tampon RIPA (additionné d'inhibiteurs de phosphatases et de protéases). 20 µg de lysat protéique sont séparés par SDS-PAGE et transférées sur membranes PVDF, qui seront ultérieurement bloquées avec du TBS-Tween 0,1% - lait écrémé 5%. Les anticorps primaires utilisés sont : Galéctine-3 (abcam ab2785), Intégrine β **1** (Millipore AB 1952), Intégrine α6 (abcam ab75737), FAK (abcam ab40794), phospo-FAK Y397 (abcam ab 80298), Talin1/2 (abcam ab11188), calpaine-2 (abcam ab155666) et GAPDH comme contrôle (Millipore MAB374). La peroxydase de raifort couplée aux anticorps secondaires est incubée 2h à température ambiante. L'imageur Chemidoc XRS+ est utilisé pour la détection de la chimioluminescence. Les quantifications de pixels sont effectuées avec le logiciel Image Lab.

### Caractérisation de la migration cellulaire

La direction et la distance de migration des cellules Gli4 sont analysées sur des fibres alignées ou non alignées, recouvertes ou non avec de la laminine. A T=0, des neurosphères de 5 000 cellules sont ensemencées. Le nombre de cellules par distance de migration est quantifié avec le logiciel AXIO-IMAJEUR. En bref, des arcs concentriques sont définis depuis le centre de la sphère à intervalles réguliers et le nombre de cellules présentes entre 2 arcs successifs est compté, définissant ainsi un nombre de cellules par intervalle de distance de migration. Les arcs de migrations sont compris entre 200 et 2 000 µm depuis le bord de la neurosphère. Cet espace est choisi pour exclure la migration liée à l'expansion de croissance des NS. Ce test est réalisé en 2D et en 3D.

### Analyse statistique

Au moins 3 réplicats sont réalisés par expérience. Les valeurs sont exprimées comme moyenne +/- SD. Les tests statistiques sont faits avec le logiciel Graphpad.

### 2.2. Résultats

### • Les fibres alignées constituent un environnement 3D pour l'adhésion et la migration de cellules de gliome

Le réseau de fibres permet de mieux comprendre, caractériser et cibler les cellules migrant dans le corps calleux. Lorsqu'elles sont déposées sur une surface 2D et cultivées en l'absence de facteurs de croissance et en présence de sérum (milieu de différentiation), les neurosphères (NS) adhèrent au support et les cellules Gli4 se différentient et migrent en s'éloignant des NS (Guichet et al "Cell death and neuronal differentiation of glioblastoma stem-like cells induced by neurogenic transcription factors", Glia. Feb;61(2):225-39, 2013). Quand elles sont ensemencées sur le réseau 3D de fibres et cultivées dans le milieu de différentiation, les NS Gli4 adhèrent à la surface et pénètrent dans le réseau de fibres (figure 2A). Les cellules Gli4 prolifèrent et migrent en s'éloignant des NS (figures 2A-2C). L'analyse de la répartition des cellules Gli4 au sein du réseau de fibres montre que les cellules migrent profondément à l'intérieur du réseau (figure 2D). Dans cet environnement, les cellules Gli4 forment des extensions cellulaires dans différentes directions pour s'attacher à plusieurs fibres, d'une manière tridimensionnelle (figures 2E et 2F). Les cellules Gli4 ont un diamètre d'environ 40 fois celui des fibres (20 µm versus environ 0,5 µm) et encerclent les fibres pour adhérer et migrer (figures 2E et 2F, flèches). Cet encerclement permet aux cellules Gli4 d'interagir de façon ventrale, latérale et dorsale au sein du réseau. De plus, les interstices hétérogènes entre les fibres se distribuent entre 0,1 et 10 µm², avec une distribution maximale entre 1 et 2 µm². Ces espaces forcent les cellules à se déformer afin de pénétrer dans le réseau de fibres (figure 2D), ce qui rappelle le confinement cellulaire naturel observé *in vivo* (Friedl and Alexander « Cancer invasion and the microenvironment : plasticity and recoiprocity », Cell, 147, 992-1009, 2011). Ces résultats montrent que les fibres de PAN sont permissives pour l'adhésion et la migration des cellules de glioblastome dans un microenvironnement 3D.

### • Dynamique de l'adhésion cellulaire aux protéines de la matrice extracellulaire et de l'adhésion focale des cellules Gli4 vis-à-vis de surfaces conventionnelles planes (2D) et de fibres alignées (3D)

Afin de comparer les interactions entre les cellules Gli4 avec le milieu extracellulaire (ECM) et leur adhésion focale (AF) sur les surfaces planes et dans le réseau 3D de fibres, fonctionnalisé ou non avec de la laminine (+ ou - LN), l'expression de l'intégrine β1, de l'intégrine a**6** et de la galectine-3 a été analysée. Entre les plaques recouvertes de laminine (PS + LN) et les fibres recouvertes de laminine (NF + LN), le niveau d'expression de la galectine-3 et de l'intégrine β1 a été augmenté de 6 et 2,6 fois, respectivement, et le niveau de l'intégrine a6 a été diminué de 2,5 fois. Entre les surfaces planes et les réseaux non recouverts de laminine, aucune différence entre les niveaux d'expression des intégrines β1 et a6 n'est observé. Par immunofluorescence, l'intégrine β1 est localisée à la membrane des cellules Gli4 migrant sur le réseau 3D recouvert de laminine, alors qu'elle est distribuée dans le cytoplasme et autour du noyau dans les cellules Gli4 déposées sur les surfaces planes recouvertes de laminine. De plus, sur les réseaux de fibres recouverts de laminine, la coloration de l'intégrine β1 est localisée aux points d'attachement avec les fibres. De plus, la phosphorylation de la kinase d'adhésion focale (FAK), un acteur majeur de la transduction du signal médié par l'intégrine, augmente lorsque les surfaces planes ou le réseau 3D sont recouverts de laminine.

L'expression de la taline 1/2, de la vinculine et de la calpaine-2 dans les cellules Gli4 sur les supports PS+LN et NF+LN a été mesurée. Les formes intactes et clivées ont été détectées dans les cellules Gli4 sur les surfaces planes avec et sans laminine, avec le plus fort taux de clivage de la taline sur le support PS+LN. Au contraire, la taline 1/2 n'est pas clivée dans les cellules Gli4 sur des fibres, avec ou sans laminine. Le niveau d'expression de la vinculine est invariant dans les différentes conditions. L'expression de la calpaine 2 est diminuée d'un facteur 2 pour les cellules Gli4 déposées sur les fibres recouvertes de laminine par rapport aux surfaces planes recouvertes de laminine.

Ces résultats montrent que l'adhésion médiée par l'intégrine β1 et la galectine-3 à ECM est augmentée sur les fibres électrofilées. La dynamique de l'adhésion focale est régulée par l'expression de la calpaine-2 et le clivage de la taline est différent entre la surface plane et le réseau 3D.

### • L'adhérence des cellules GliT et leur interaction avec les cellules de la matrice extracellulaire ne sont pas améliorées sur les fibres

Les cellules GliT sont une autre lignée primaire de cellules de neuroblastome, moins invasive que Gli4 in vivo. GliT et Gli4 diffèrent pour l'expression de nombreuses protéines en lien avec la matrice extracellulaire, en particulier l'expression de la galectine-3, l'intégrine β1 et l'intégrine a6 diffèrent, dans les différents systèmes étudiés (surface plane ou neurofibres, avec ou sans laminine). L'adhésion des cellules GliT via la galectine-3, l'intégrine β1 et l'intégrine a6 n'est pas améliorée en présence du réseau de fibres.

### • L'intégrine β1 et la galectine-3 sont surexprimées dans les cellules Gli4 dans le corps calleux alors que la calpaine-2 est sous-exprimée dans les cellules Gli4 et GliT invasives in vivo

Les résultats obtenus indiquent que les cellules Gli4 sont plus invasives que les cellules GliT, et que l'expression de l'intégrine β1 et de la galectine-3 dans les cellules Gli4 est dépendante du microenvironnement cérébral et que l'expression de la calpaine-2 semble inversement corrélée au potentiel invasif des cellules invasives de gliobastome (GICs) in vivo.

### • Les cellules Gli4 migrent individuellement ou collectivement en présence ou en absence de laminine sur des fibres alignées de PAN.

Comme observé en microscopie en fluorescence et de reconstitution d'image en 3D de microscopie électronique (Figures 3A à 3D), lorsqu'elles sont ensemencées sur un réseau 3D de fibres non recouvert de laminine, les cellules Gli4 migrent collectivement en formant des agrégats composés de cellules fortement associées entre elles. Au contraire, en présence d'un réseau de fibres recouvert de laminine, les cellules Gli4 se séparent les unes des autres et migrent de manière individuelle. Les cellules adoptent donc deux modes différents de migration, mode collectif ou mode individuel, selon la fonctionnalisation des fibres. De plus, sur les fibres « sans laminine », la coloration à la phalloïdine de l'actine du cytosquelette montre que la plupart des cellules sont rondes, au sein des agrégats le cytosquelette d'actine est continu entre les cellules, au centre de la masse cellulaire les cellules sont rondes alors qu'à l'extérieur de l'agrégat elles sont bipolaires et ont un lamellipode. Au contraire, sur le réseau de fibres recouvert de laminine, les cellules Gli4 sont individuelles, bipolaires, asymétriques et chacune possède un lamellipode sur le côté avant. De plus, les cellules expriment la N-cadhérine sur leur membrane.

Par ailleurs, le nombre d'événements de migration collective par neurosphères a été quantifié, sur le réseau 3D fonctionnalisé ou non avec la laminine. Pour cela, des neurosphères de la même taille et contenant le même nombre de cellules ont été ensemencées sur les deux réseaux. Une diminution significative (^{∗∗∗∗}p<0,0001) du nombre de migrations collectives par neurosphères est observé après la fonctionnalisation des fibres avec la laminine. Le nombre d'unités de migration collective par neurosphère décroit de 80 à 20 lorsque les fibres sont fonctionnalisées.

### • Les cellules Gli4 répondent à un signal de topoinduction résultant de l'orientation et de l'organisation des fibres electrofilées biocompatibles.

La direction de la migration des cellules Gli4 sur les fibres alignées (AF) ou non-alignées (N-AF), fonctionnalisées ou non par la laminine, est comparé. Les résultats montrent que la présence de laminine augmente le nombre de cellules migratrices à l'extérieur des NS, sur les fibres alignées et non-alignées. Sur les fibres non alignées, les cellules Gli4 migrent en s'éloignant des neurosphères dans toutes les directions, en absence ou en présence de laminine. Sur les fibres alignées, les cellules Gli4 migrent de façon prédominante dans la direction des fibres, plutôt que dans la direction perpendiculaire, et cet effet est observé principalement en présence de laminine. Le nombre de cellules migratoires, en fonction de la distance de migration sur les fibres a été quantifié. La longueur de la région de migration a été délimitée entre la bordure des neurosphères et une distance de 2 mm dans la direction de la migration. Les résultats montrent une augmentation significative du nombre de cellules migratoires sur les fibres non alignées en présence de laminine (histogramme de droite), par comparaison avec les fibres non alignées sans laminine (histogramme de gauche) (Fig. 4). L'addition de laminine augmente le nombre de cellules Gli4 migratoires en direction parallèle aux fibres alignées, et en direction perpendiculaire. En direction parallèle des fibres et en présence de laminine, le nombre de cellules Gli4 augmente significativement à une distance entre 200 et 800 µm du bord des NS, par comparaison avec les fibres non fonctionnalisées. Le même résultat est observé dans la direction perpendiculaire aux fibres alignées, dans lequel le nombre de cellules augmente aussi significativement à une distance entre 200 et 600 µm du bord des NS en présence de laminine. Au contraire, aucune différence significative n'est observée dans le nombre de cellule migratoires près du bord des NS (de 0 à 200 µm), en parallèle et perpendiculairement aux fibres, en présence et en absence de laminine. La présence de cellules adjacentes aux NS résulte de prolifération plus que de migration. Une augmentation significative du nombre de cellules migratoires en direction parallèle des fibres alignes est observé par rapport à la direction perpendiculaire, en absence et en présence de laminine. Dans le modèle décrit, il n'est donc pas nécessaire que les fibres soient alignées pour créer des espaces interstitiels permissifs pour l'infiltration cellulaire.

L'ensemble de ces résultats montre que la présence de laminine augmente la migration de cellules Gli4 sur les fibres alignées et non alignées. De plus l'orientation des fibres dicte la direction de la migration des cellules Gli4. Sur les fibres non orientées, les cellules Gli4 migrent dans toutes les directions, alors que sur les fibres alignées elles migrent davantage dans une direction parallèle aux fibres que dans une direction perpendiculaire.

En conclusion, la matrice 3D de fibres électrofilées permet l'adhésion de cellules de glioblastome et leur migration dans un microenvironnement 3D. Ce modèle montre l'importance de l'adhésion cellule-cellule via la N-cadhérine dans le processus de migration collective et le rôle de la laminine dans la migration accélérée de cellules de glioblastome. Cette matrice de fibres représente un outil important notamment pour étudier le rôle de la matrice extra-cellulaire dans la migration des cellules de glioblastome.

### EXEMPLE 3 : SYNTHESE DE RESEAUX 3D DE FIBRES DE POLYMERES DE TYPE ACRYLIQUE ET CARACTERISATION PHYSIQUE

### 3.1. Matériels et méthodes

### 3.1.1. Réseau 3D de fibres alignées de polymère PMMA 5%, PAN 5%

Une solution à 5% massique de Polyacrylonitrile (Sigma Aldrich) et 5% de poly(méthacrylate de méthyle) (PMMA, Sigma-Aldrich) est préparée dans du diméthylformamide. Pour 10 g de solution de polymères : pesée à valeur de 0,5 g de PAN et 0,5 g de PMMA pour 9 g de DMF, puis chauffage à 75°C avec agitation jusqu'à dissolution complète des polymères dans le DMF.

Cette solution est mise en forme par électrofilage. Le collecteur est un collecteur rotatif de 15 cm de diamètre. Un champ électrique de 20 kV est appliqué entre l'aiguille distribuant le polymère et le collecteur. L'aiguille se trouve à une distance de 15 cm du collecteur rotatif. Le collecteur autour duquel s'enroulent les fibres possède une vitesse de rotation de 2.000 rpm permettant d'obtenir un réseau dont les fibres sont alignées. Le polymère est acheminé jusqu'à l'aiguille grâce à un « pousse-seringue » avec une force de poussée de 3,4 ml/h dans le cas présent. Durant l'électrofilage, l'aiguille réalise des déplacements d'une amplitude de 40 mm avec une vitesse de déplacement de 5 mm/sec. L'électrofilage dure au moins 20 minutes. Le tissu de fibres subit ensuite un traitement thermique avant d'être stérilisé.

Le réseau obtenu par électrofilage est traité thermiquement sous air à une température de 110 °C, avec une vitesse de chauffe de 120 °C/heure, un palier à 110 °C pendant 1 heure, la vitesse de refroidissement est de 300 °C/h. Lors du traitement, la couleur du réseau passe de blanc à marron, traduisant l'aromatisation des fibres.

Avant utilisation, le réseau subit une stérilisation à l'éthanol 70° ou à l'autoclave.

### 3.1.2. Réseau 3D de fibres de polymères PMMA 5%, PAN 10%

Une solution à 10% massique de Polyacrylonitrile (Sigma Aldrich) et 5% de poly(méthacrylate de méthyle) (PMMA, Sigma-Aldrich) est préparée dans du diméthylformamide. Pour 10 g de solution de polymères : pesée à valeur de 1,0 g de PAN et 0,5 g de PMMA pour 8,5 g de DMF, puis chauffage à 75°C avec agitation jusqu'à dissolution complète des polymères dans le DMF.

Cette solution est mise en forme par électrofilage. Le collecteur est un collecteur rotatif de 15 cm de diamètre. Un champ électrique de 20 kV est appliqué entre l'aiguille distribuant le polymère et le collecteur. L'aiguille se trouve à une distance de 15 cm du collecteur rotatif. Le collecteur autour duquel s'enroulent les fibres possède une vitesse de rotation de 2.000 rpm permettant d'obtenir un réseau dont les fibres sont alignées. Le polymère est acheminé jusqu'à l'aiguille grâce à un « pousse-seringue » avec une force de poussée de 2,4 ml/h dans le cas présent. Durant l'électrofilage, l'aiguille réalise des déplacements d'une amplitude de 40 mm avec une vitesse de déplacement de 5 mm/sec. L'électrofilage dure au moins 20 minutes. Le tissu de fibres subit ensuite un traitement thermique avant d'être stérilisé.

Le réseau obtenu par électrofilage est traité thermiquement sous air à une température de 110 °C, avec une vitesse de chauffe de 120 °C/heure, un palier à 110 °C pendant 1 heure, la vitesse de refroidissement est de 300 °C/h. Lors du traitement, la couleur du réseau passe de blanc à marron, traduisant l'aromatisation des fibres.

Avant utilisation, le réseau subit une stérilisation à l'éthanol 70° ou à l'autoclave.

### 3.1.3. Réseau 3D de fibres de polymères PMMA 20%, +DMF +THF

Une solution à 20% massique de poly(méthacrylate de méthyle) (PMMA, Sigma-Aldrich) est préparée dans un mélange de diméthylformamide et de tétrahydrofurane (THF). Pour 10 g de solution de polymères : pesée à valeur de 2,0 g de PMMA pour 4 g de DMF et 4 g de THF, puis agitation jusqu'à dissolution complète des polymères dans le solvant.

Cette solution est mise en forme par électrofilage. Le collecteur est un collecteur rotatif de 15 cm de diamètre. Un champ électrique de 20 kV est appliqué entre l'aiguille distribuant le polymère et le collecteur. L'aiguille se trouve à une distance de 15 cm du collecteur rotatif. Le collecteur autour duquel s'enroulent les fibres possède une vitesse de rotation de 2.000 rpm permettant d'obtenir un réseau dont les fibres sont alignées. Le polymère est acheminé jusqu'à l'aiguille grâce à un « pousse-seringue » avec une force de poussée de 3,8 ml/h dans le cas présent. Durant l'électrofilage, l'aiguille réalise des déplacements d'une amplitude de 40 mm avec une vitesse de déplacement de 5 mm/sec. L'électrofilage dure au moins 20 minutes. Le tissu de fibres subit ensuite un traitement thermique avant d'être stérilisé.

Le réseau obtenu par électrofilage est traité thermiquement sous air à une température de 110 °C, avec une vitesse de chauffe de 120 °C/heure, un palier à 110 °C pendant 1 heure, la vitesse de refroidissement est de 300 °C/h. Lors du traitement, la couleur du réseau passe de blanc à marron, traduisant l'aromatisation des fibres.

Avant utilisation, le réseau subit une stérilisation à l'éthanol 70° ou à l'autoclave.

### 3.2. Résultats

### Caractérisation des réseaux 3D de fibres de polymère

Les Figures 7A à 7C représentent les réseaux 3D selon l'invention obtenus par la réticulation des solutions de polymère comprenant respectivement :
- 5% PMMA, 5% PAN dans du DMF : Fig. 7A
- 5% PMMA, 10% PAN dans du DMF : Fig. 7B
- 20% PMMA, dans du DMF + THF : Fig. 7C

Les images de microscopie électronique à balayage montrent que le diamètre moyen des fibres est de 300 nm dans le cas du mélange « 5% PMMA, 5% PAN », 600 nm dans le cas du mélange « 5% PMMA, 10% PAN » et de 1,2 µm dans le cas du « 20% PMMA ».

### EXEMPLE 4 : CARACTERISATION DE LA VIABILITE DE CELLULES DE GLIOBLASTOME

### 4.1. Matériel et méthodes

### Culture de cellules de glioblastome Gli4

### Culture de cellules sur le réseau 3D

Avant le dépôt des cellules, les réseaux 3D de fibres electrofilées biocompatibles (désignées par : « fibres »), préparées comme décrit dans l'exemple 3 sont stérilisés en alcool à 70% autoclavé. Les plaques et les fibres sont lavées 2 fois en eau stérile.

Pour obtenir des neurosphères, des microplaques Corning à 96 puits sont utilisées (Corning 7007). Les neurosphères dissociées de Gli4 sont cultivées en conditions de prolifération, et ensemencées à 5.000 cellules par puits. Les cellules sédimentent et les NS sont récoltées 24 heures après ensemencement.

### 4.2. Résultats

La figure 8 (test MTT) est un histogramme illustrant en ordonnées l'absorbance du formazan produit par réduction du sel de tétrazolium MTT (bromure de 3-(4,5-dimethylthiazol-2-yl)-2,5-diphenyl tetrazolium) par les cellules vivantes et actives métaboliquement sur les fibres de polymère, en abscisses, de gauche à droite, PAN 10% ; PMMA 5% + PAN 5% ; PMMA 5%+ PAN 10% et PMMA 20%. Ce test MTT, bien connu de l'homme du métier, montre que le réseau 3D constitué de fibres dérivées d'acryliques ou obtenues à partir de mélange de polymères de type acrylique ne montre pas de cytotoxicité. Ce test a été réalisé sur 30 000 cellules Gli4 déposées en dissociées sur chaque réseau de fibres. Le temps d'incubation est de 5 jours en milieu de différenciation.

### EXEMPLE 5: CARACTERISATION DE LA MIGRATION DE CELLULES DE GLIOBLASTOME

### 5.1. Matériel et méthodes

Avant le dépôt des cellules, les réseaux 3D de fibres electrofilées biocompatibles (désignées par : « fibres »), préparées comme décrit dans l'exemple 3 sont stérilisés en alcool à 70% autoclavé. Les plaques et les fibres sont lavées 2 fois en eau stérile.

Pour obtenir des neurosphères, des microplaques Corning à 96 puits sont utilisées (Corning 7007). 7500 cellules Gli4 sont déposées dans chaque puit et cultivées en conditions de prolifération de sorte à former des neurosphères de Gli4 de même taille. Les NS sont récoltées 48 heures après ensemencement et déposées sur les différentes fibres. Les neurosphères sont laissées 5 jours en migration dans un milieu de différenciation avant fixation et coloration tel que décrit dans l'exemple 1.

### 5.2. Résultats

Les images de microscopie à fluorescence de Gli4 en neurosphères de même taille (7500 cellules) ensemencées sur les fibres dérivées d'acrylique ou obtenues à partir de mélange de polymères de type acrylique montrent une permissivité desdits réseaux à la migration cellulaire ainsi qu'une adhérence des cellules. Les Gli4 migrent parallèlement à l'orientation préférentielle du réseau de fibres. Le cytosquelette d'actine des Gli4 est marqué à la phalloïdine et le noyau est marqué au Hoechst.

Les aires de migrations sont ensuite calculées grâce au logiciel zen, en soustrayant l'aire initiale de la neurosphère, permettant d'évaluer l'amplitude la migration sur les fibres. Les résultats (Figures 9A à 9C, Figure 10) montrent l'influence des caractéristiques physiques et chimiques des fibres sur la cinétique de migration.

## Revendications

1. Réseau tridimensionnel infusible de fibres de polymère de type acrylique réticulé, **caractérisé en ce que** :
- le diamètre desdites fibres est compris entre 0,1 et 1,5 µm, de préférence entre 0,3 et 1 µm, et plus préférentiellement entre 0,6 et 0,8 µm,
- la taille des interstices entre lesdites fibres est comprise entre 0,1 et 50 µm², de préférence entre 0,5 et 10 µm², et plus préférentiellement entre 1 et 2 µm², et
- la rigidité dudit réseau est **caractérisée par** un module d'élasticité compris entre 0,01 et 10 000 kPa, de préférence entre 0,1 et 10 000 kPa, de préférence entre 0,1 et 1 000 kPa, plus préférentiellement entre 0,1 et 300 kPa.

2. Réseau tridimensionnel selon la revendication 1, **caractérisé en ce que** :
- ledit polymère est obtenu par la réticulation thermique de polyacrylonitrile (PAN),
- la rigidité du réseau est **caractérisée par** un module d'élasticité compris entre 0,01 et 10 000 kPa, de préférence entre 0,1 et 10 000 kPa, de préférence entre 0,1 et 1 000 kPa, plus préférentiellement entre 0,1 et 300 kPa, et
- optionnellement la surface des fibres est recouverte par au moins une protéine de la matrice extracellulaire, de préférence choisie parmi : la laminine, la fibronectine, la vitronectine, l'acide hyaluronique et le collagène.

3. Réseau tridimensionnel selon la revendication 2, **caractérisé en ce que** :
- ledit polymère est obtenu par la réticulation thermique de PAN,
- ledit polymère réticulé comprend des nanocharges, de préférence des nanotubes de carbone, à une concentration comprise entre 0,00001 et 5 %, de préférence entre 0,00001 et 1 %, de préférence entre 0,0001 et 0,1 %, de préférence entre 0,0002 et 0,08 %, de préférence entre 0,00075 et 0,05 %, ledit pourcentage étant exprimé en pourcentage massique de la solution du polymère avant réticulation,
- la rigidité du réseau est **caractérisée par** un module d'élasticité compris entre 0,1 et 1 000 kPa, de préférence entre 0,1 et 300 kPa, et
- la surface des fibres est recouverte de laminine.

4. Procédé de préparation d'un réseau tridimensionnel de fibres de polymère réticulé selon la revendication 3, ledit procédé comprenant les étapes successives suivantes :
a) une étape de synthèse dudit réseau par électrofilage d'une solution de PAN dont la concentration est comprise entre 8 et 12 %, de préférence de 10 %, exprimé en pourcentage massique de ladite solution de PAN, pour obtenir un réseau tridimensionnel de fibres, et
b) une étape de traitement thermique sous atmosphère oxydante et à une température comprise entre 40 °C et 400 °C, de préférence entre 200 °C et 300 °C, du réseau tridimensionnel de fibres obtenu lors de l'étape a),
ladite étape de synthèse par électrofilage étant **caractérisée en ce que** :
- la solution de PAN est projetée par une aiguille dont l'amplitude de déplacement est comprise entre 30 et 50 mm, de préférence 40 mm, et dont la vitesse de déplacement est comprise entre 2 et 10 mm/seconde, de préférence 5 mm/seconde,
- la distance entre la source de polymère et l'électrode collectrice est comprise entre 1 et 50 cm, de préférence entre 10 et 30 cm et plus préférentiellement 15 cm, et le champ électrique appliqué dans le champ de projection est compris entre 16 et 24 kV, de préférence 20 kV, et
- le débit de la solution de polymère lors de l'alimentation de la seringue est compris entre 0,5 et 8,6 mL/h.

5. Procédé selon la revendication 4, **caractérisé en ce que** la solution de PAN est projetée sur une électrode collectrice rotative, de préférence un tambour, de diamètre compris entre 12 et 18 cm, de préférence de 15 cm, la vitesse de rotation de ladite électrode étant comprise entre 1 et 100 000 g, de préférence entre 1 et 1 000 g et plus préférentiellement de 335 g.

6. Procédé selon l'une quelconque des revendications 4 ou 5, **caractérisé en ce que** ladite solution de polymère comprend des nanotubes de carbone à une concentration comprise entre 0,00001 et 5 %, de préférence entre 0,00001 et 1 %, de préférence entre 0,0001 et 0,1 %, de préférence entre 0,0002 et 0,08 %, de préférence entre 0,00075 et 0,05 %, exprimé en pourcentage massique de ladite solution de polymère avant réticulation.

7. Procédé selon l'une quelconque des revendications 4 à 6, **caractérisé en ce que** ladite étape de traitement thermique est suivie par une étape de traitement de la surface desdites fibres, comprenant la mise en présence dudit réseau avec une solution d'au moins une protéine de la matrice extracellulaire, ladite protéine étant de préférence choisie parmi : la laminine, la fibronectine, la vitronectine, l'acide hyaluronique et le collagène, ladite mise en présence étant optionnellement précédée par un traitement préalable de la surface des fibres dudit réseau.

8. Dispositif pour le support de cellules, comprenant au moins un réseau selon l'une quelconque des revendications 1 à 3 ou un réseau susceptible d'être obtenu par un procédé selon l'une quelconque des revendications 4 à 7.

9. Utilisation d'un réseau tridimensionnel selon l'une quelconque des revendications 1 à 3, d'un réseau tridimensionnel obtenu par un procédé selon l'une quelconque des revendications 4 à 7 ou d'un dispositif selon la revendication 8, en tant que support de cellules.

10. Utilisation selon la revendication 9, **caractérisée en ce qu'**elle comprend les étapes successives suivantes :
- la mise en contact de cellules avec un réseau tridimensionnel selon l'une des revendications 1 à 3, un réseau tridimensionnel obtenu par un procédé selon l'une quelconque des revendications 4 à 7 ou un dispositif selon la revendication 8, et
- l'observation et optionnellement la détermination d'au moins un paramètre caractérisant lesdites cellules et/ou l'analyse biochimique desdites cellules.

## Patentansprüche

1. Dreidimensionales nichtverschmelzendes Netzwerk aus Polymerfasern vom vernetzten Acryltyp, **dadurch gekennzeichnet, dass**:
- der Durchmesser dieser Fasern im Bereich von 0,1 bis 1,5 µm, vorzugsweise von 0,3 bis 1 µm, und besonders bevorzugt von 0,6 bis 0,8 µm, liegt,
- die Größe von Zwischenräumen zwischen diesen Fasern im Bereich von 0,1 bis 50 µm², vorzugsweise von 0,5 bis 10 µm², und besonders bevorzugt von 1 bis 2 µm², liegt, und
- die Steifigkeit des Netzwerks durch einen Elastizitätsmodul gekennzeichnet ist, der im Bereich von 0,01 bis 10.000 kPa, vorzugsweise von 0,1 bis 10.000 kPa, bevorzugt von 0,1 bis 1.000 kPa, besonders bevorzugt von 0,1 bis 300 kPa, liegt.

2. Dreidimensionales Netzwerk nach Anspruch 1, **dadurch gekennzeichnet, dass**:
- das Polymer durch thermische Vernetzung von Polyacrylnitril (PAN) erhalten ist,
- die Steifigkeit des Netzwerks durch einen Elastizitätsmodul gekennzeichnet ist, der im Bereich von 0,01 bis 10.000 kPa, vorzugsweise von 0,1 bis 10.000 kPa, bevorzugt von 0,1 bis 1.000 kPa, besonders bevorzugt von 0,1 bis 300 kPa, liegt, und
- optional die Oberfläche der Fasern mit mindestens einem Protein der extrazellulären Matrix bedeckt ist, vorzugsweise ausgewählt aus: Laminin, Fibronectin, Vitronectin, Hyaluronsäure und Kollagen.

3. Dreidimensionales Netzwerk nach Anspruch 2, **dadurch gekennzeichnet, dass**:
- das Polymer durch thermische Vernetzung von PAN erhalten ist,
- das vernetzte Polymer Nanofüllstoffe, vorzugsweise Kohlenstoffnanoröhren, in einer Konzentration im Bereich von 0,00001 bis 5 %, vorzugsweise von 0,00001 bis 1 %, vorzugsweise von 0,0001 bis 0,1 %, vorzugsweise von 0,0002 bis 0,08 %, vorzugsweise von 0,00075 bis 0,05%, beinhaltet, wobei dieser Prozentsatz als Masseprozent der Polymerlösung vor der Vernetzung ausgedrückt ist,
- die Steifigkeit des Netzwerks durch einen Elastizitätsmodul gekennzeichnet ist, der im Bereich von 0,1 bis 1.000 kPa, vorzugsweise von 0,1 bis 300 kPa, liegt, und
- die Oberfläche der Fasern mit Laminin beschichtet ist.

4. Verfahren zur Herstellung eines dreidimensionalen Netzwerks aus vernetzten Polymerfasern nach Anspruch 3, wobei das Verfahren die folgenden aufeinanderfolgenden Schritte umfasst:
a) einen Schritt der Erzeugung des Netzwerks durch Elektrospinnen einer PAN-Lösung, deren Konzentration im Bereich von 8 bis 12 %, vorzugsweise 10 %, ausgedrückt als Masseprozent der PAN-Lösung, liegt, um ein dreidimensionales Netzwerk von Fasern zu erhalten, und
b) einen Schritt der Wärmebehandlung in einer oxidierenden Atmosphäre und bei einer Temperatur im Bereich von 40 °C bis 400 °C, vorzugsweise von 200 °C bis 300 °C, des in Schritt a) erhaltenen dreidimensionalen Netzwerks aus Fasern,
wobei der Schritt der Erzeugung durch Elektrospinnen **dadurch gekennzeichnet ist, dass**:
- die PAN-Lösung von einer Spritznadel verspritzt wird, deren Bewegungsamplitude im Bereich von 30 bis 50 mm, vorzugsweise 40 mm, liegt, und deren Bewegungsgeschwindigkeit im Bereich von 2 bis 10 mm/Sekunde, vorzugsweise 5 mm/Sekunde, liegt,
- der Abstand zwischen der Polymerquelle und der Sammelelektrode im Bereich von 1 bis 50 cm, vorzugsweise von 10 bis 30 cm, und besonders bevorzugt 15 cm, liegt, und das im Spritzfeld angelegte elektrische Feld im Bereich von 16 bis 24 kV, vorzugsweise 20 kV, liegt, und
- die Durchflussrate der Polymerlösung bei der Zufuhr zur Spritze im Bereich von 0,5 bis 8,6 mL/h liegt.

5. Verfahren nach Anspruch 4, **dadurch gekennzeichnet, dass** die PAN-Lösung auf eine rotierende Sammelelektrode, vorzugsweise eine Trommel, mit einem Durchmesser im Bereich von 12 bis 18 cm, vorzugsweise 15 cm, gespritzt wird, wobei die Rotationsgeschwindigkeit dieser Elektrode im Bereich von 1 bis 100.000 g, vorzugsweise 1 bis 1.000 g, besonders bevorzugt 335 g, liegt.

6. Verfahren nach einem der Ansprüche 4 oder 5, **dadurch gekennzeichnet, dass** die Polymerlösung Kohlenstoffnanoröhren in einer Konzentration enthält, die im Bereich von 0,00001 bis 5 %, vorzugsweise 0,00001 bis 1 %, vorzugsweise 0,0001 bis 0,1 %, vorzugsweise 0,0002 bis 0,08 %, bevorzugt 0,00075 bis 0,05 %, ausgedrückt als Masseprozent der Polymerlösung vor der Vernetzung, liegt.

7. Verfahren nach einem der Ansprüche 4 bis 6, **dadurch gekennzeichnet, dass** auf den Schritt der Wärmebehandlung ein Schritt der Behandlung der Oberfläche der Fasern folgt, der das Zusammenbringen des Netzwerks mit einer Lösung von mindestens einem Protein der extrazellulären Matrix umfasst, wobei das Protein vorzugsweise ausgewählt ist aus: Laminin, Fibronectin, Vitronectin, Hyaluronsäure und Kollagen, wobei dem Zusammenbringen optional eine Vorbehandlung der Oberfläche der Fasern des Netzwerks vorausgegangen ist.

8. Vorrichtung zum Tragen von Zellen, aufweisend mindestens ein Netzwerk nach einem der Ansprüche 1 bis 3 oder ein Netzwerk, das durch ein Verfahren nach einem der Ansprüche 4 bis 7 erhalten werden kann.

9. Verwendung eines dreidimensionalen Netzwerks nach einem der Ansprüche 1 bis 3, eines dreidimensionalen Netzwerks, das durch ein Verfahren nach einem der Ansprüche 4 bis 7 erhalten werden kann, oder einer Vorrichtung nach Anspruch 8, als Träger von Zellen.

10. Verwendung nach Anspruch 9, **dadurch gekennzeichnet, dass** sie die folgenden aufeinanderfolgenden Schritte umfasst:
- In-Kontakt-Bringen von Zellen mit einem dreidimensionalen Netzwerk nach einem der Ansprüche 1 bis 3, einem dreidimensionalen Netzwerk, das durch ein Verfahren nach einem der Ansprüche 4 bis 7 erhalten wurde, oder einer Vorrichtung nach Anspruch 8, und
- Beobachtung und optional Bestimmung mindestens eines Parameters, der die Zellen charakterisiert, und/oder biochemische Analyse der Zellen.

## Claims

1. Infusible three-dimensional network of crosslinked acrylic-type polymer fibres, **characterized in that**:
- the diameter of said fibres is comprised between 0.1 and 1.5 µm, preferably between 0.3 and 1 µm, and more preferentially between 0.6 and 0.8 µm,
- the size of the interstices between said fibres is comprised between 0.1 and 50 µm², preferably between 0.5 and 10 µm², and more preferentially between 1 and 2 µm², and
- the stiffness of said network is **characterized by** an elastic modulus comprised between 0.01 and 10,000 kPa, preferably between 0.1 and 10,000 kPa, preferably between 0.1 and 1,000 kPa, more preferentially between 0.1 and 300 kPa.

2. Three-dimensional network according to claim 1, **characterized in that**:
- said polymer is obtained by thermal crosslinking of polyacrylonitrile (PAN),
- the stiffness of the network is **characterized by** an elastic modulus comprised between 0.01 and 10,000 kPa, preferably between 0.1 and 10,000 kPa, preferably between 0.1 and 1,000 kPa, more preferentially between 0.1 and 300 kPa, and
- optionally, the surface of the fibres is coated with at least one protein of the extracellular matrix, preferably selected from: laminin, fibronectin, vitronectin, hyaluronic acid and collagen.

3. Three-dimensional network according to claim 2, **characterized in that**:
- said polymer is obtained by thermal crosslinking of PAN,
- said crosslinked polymer comprises nanofillers, preferably carbon nanotubes, at a concentration comprised between 0.00001 and 5%, preferably between 0.00001 and 1%, preferably between 0.0001 and 0.1%, preferably between 0.0002 and 0.08%, preferably between 0.00075 and 0.05%, said percentage being expressed in weight percent of the polymer solution before crosslinking,
- the stiffness of the network is **characterized by** an elastic modulus comprised between 0.1 and 1,000 kPa, preferably between 0.1 and 300 kPa, and
- the surface of the fibres is coated with laminin.

4. Process for preparing a three-dimensional network of crosslinked polymer fibres according to claim 3, said process comprising the following successive steps:
a) a step of synthesizing said network by electrospinning of a PAN solution the concentration of which is comprised between 8 and 12%, preferably 10%, expressed in weight percent of said PAN solution, in order to obtain a three-dimensional network of fibres, and
b) a step of heat treatment under oxidizing atmosphere and at a temperature comprised between 40°C and 400°C, preferably between 200°C and 300°C, of the three-dimensional network of fibres obtained during step a),
said step of synthesis by electrospinning being **characterized in that**:
- the PAN solution is extruded from a needle the displacement amplitude of which is comprised between 30 and 50 mm, preferably 40 mm, and the displacement rate of which is comprised between 2 and 10 mm/second, preferably 5 mm/second,
- the distance between the polymer source and the collecting electrode is comprised between 1 and 50 cm, preferably between 10 and 30 cm and more preferentially 15 cm, and the electrical field applied in the extrusion field is comprised between 16 and 24 kV, preferably 20 kV, and
- the flow rate of the polymer solution during supply of the syringe is comprised between 0.5 and 8.6 mL/h.

5. Process according to claim 4, **characterized in that** the PAN solution is extruded onto a rotating collecting electrode, preferably a drum, with a diameter comprised between 12 and 18 cm, preferably 15 cm, the speed of rotation of said electrode being comprised between 1 and 100,000 g, preferably between 1 and 1,000 g and more preferentially 335 g.

6. Process according to any one of claims 4 or 5, **characterized in that** said polymer solution comprises carbon nanotubes at a concentration comprised between 0.00001 and 5%, preferably between 0.00001 and 1%, preferably between 0.0001 and 0.1%, preferably between 0.0002 and 0.08%, preferably between 0.00075 and 0.05%, expressed in weight percent of said polymer solution before crosslinking.

7. Process according to any one of claims 4 to 6, **characterized in that** said heat treatment step is followed by a step of treating the surface of said fibres, comprising bringing said network into the presence of a solution of at least one protein of the extracellular matrix, said protein being preferably selected from: laminin, fibronectin, vitronectin, hyaluronic acid and collagen, said bringing it into the presence being optionally preceded by a prior treatment of the surface of the fibres of said network.

8. Device for cell support, comprising at least one network according to any one of claims 1 to 3 or a network capable of being obtained by a process according to any one of claims 4 to 7.

9. Use of a three-dimensional network according to any one of claims 1 to 3, of a three-dimensional network obtained by a process according to any one of claims 4 to 7 or of a device according to claim 8, as a cell support.

10. Use according to claim 9, **characterized in that** it comprises the following successive steps:
- bringing cells into contact with a three-dimensional network according to one of claims 1 to 3, a three-dimensional network obtained by a process according to any one of claims 4 to 7 or a device according to claim 8, and
- observing and optionally determining at least one parameter characterizing said cells and/or the biochemical analysis of said cells
